(19) Europäisches Patentamt / European Patent Office / Office européen des brevets

(11) **EP 3 572 144 A1**

(12) **EUROPEAN PATENT APPLICATION**
published in accordance with Art. 153(4) EPC

(43) Date of publication:
**27.11.2019 Bulletin 2019/48**

(21) Application number: **17892982.4**

(22) Date of filing: **26.12.2017**

(51) Int Cl.:
*B01D 69/06* (2006.01)   *B01D 69/10* (2006.01)
*B01D 71/08* (2006.01)   *C12M 1/34* (2006.01)
*C12M 3/00* (2006.01)   *C12N 5/07* (2010.01)
*C12Q 1/06* (2006.01)

(86) International application number:
**PCT/JP2017/046650**

(87) International publication number:
**WO 2018/135252 (26.07.2018 Gazette 2018/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **18.01.2017 JP 2017006741**

(71) Applicant: **NATIONAL AGRICULTURE AND FOOD RESEARCH ORGANIZATION**
**Tsukuba-shi, Ibaraki 305-8517 (JP)**

(72) Inventor: **TAKEZAWA Toshiaki**
**Tsukuba-shi**
**Ibaraki 305-8602 (JP)**

(74) Representative: **V.O.**
**P.O. Box 87930**
**2508 DH Den Haag (NL)**

(54) **SEMIPERMEABLE MEMBRANE AND USES THEREOF**

(57)    A semipermeable membrane includes a holding body with a low water absorption property having a lattice structure and having a semipermeable property in a liquid phase. A cell-culturing device is provided with the semipermeable membrane at least at a portion thereof. A tissue-type chip is provided with the cell-culturing device including one type of cells. An organ-type chip is provided with the cell-culturing device including at least two types of cells. A kit for providing a multicellular structure is provided with an openable and closable sealed container including the tissue-type chip or the organ-type chip, and a culture medium. An organ-type chip system is provided with at least two of the tissue-type chips or the organ-type chips, and the tissue-type chips or the organ-type chips are connected while maintaining a sealing property. A cell-culturing method is a method of using the cell-culturing device. A method for measuring a number of cells is a method using the cell-culturing device.

FIG. 1

EP 3 572 144 A1

# Description

## Technical Field

The present invention relates to a semipermeable membrane, a cell-culturing device, a tissue-type chip, an organ-type chip, an organ-type chip system, a cell-culturing method using the cell-culturing device, and a cell number measurement method using the cell-culturing device.

Priority is claimed on Japanese Patent Application No. 2017-006741, filed on January 18, 2017, the content of which is incorporated herein by reference.

## Background Art

Enterprises involved in the discovery of drugs and alternative methods to animal experiments purchase frozen cells from a cell bank or the like, then cryopreserve the sub-cultured and proliferated cells to prepare culture models with some of the cells in order to conduct the tests necessary to carry out development. In other words, large amounts of money and time are consumed before the tests necessary for development are carried out.

Furthermore, "tissue-type culture models" which are closer to living bodies, rather than monolayer culture cells, are required.

Examples of techniques related to "tissue-type culture models" include, for example, various gel-embedding culturing techniques, spheroid-culturing techniques (refer to, for example, PTL 1), various chamber-culturing techniques (refer to, for example, PTL 2), and the like.

Meanwhile, the present inventors have developed a hydrogel thin membrane containing an extracellular matrix component, a chamber provided with the hydrogel thin membrane, and the like. For example, PTL 3 discloses a hydrogel thin membrane integrated with a thin membrane formed of a hydrated product of a vitrified matrix gel thin membrane containing an extracellular matrix component, and a holding body such as a circular body or a mesh body.

## Citation List

### Patent Literature

[PTL 1] Japanese Unexamined Patent Application, First Publication No. 2012-65555
[PTL 2] Republished PCT International Publication No. WO 2008/130025
[PTL 3] Japanese Unexamined Patent Publication No. 8-228768

## Summary of Invention

## Technical Problem

In the related art related to the "tissue-type culture models" described in PTLs 1 and 2, the culture operation is complicated in all cases, and not only does it take time and money to construct the tissue-type culture model, but the production reproducibility is also not always good, thus, there may be differences between lots. Furthermore, culturing techniques in which cells are exposed, such as the spheroid-culturing technique, do not always have a good protective performance for individual cells forming three-dimensional tissue. In addition, in the related art techniques relating to the above-described "tissue-type culture model", it is difficult to maintain the cells being cultured for a long period of time and there is a time restriction in that it is necessary to use the culture model immediately after construction.

In addition, in the cell-culturing device provided with the hydrogel thin membrane containing the extracellular matrix component described in PTL 3, the membrane bends when a liquid is injected therein, and there is a problem in that the membrane will be damaged if the portion of the hydrogel thin membrane is further held with tweezers or the like. In addition, in a cell-culturing device provided with a hydrogel thin membrane integrated with a holding body such as a gauze, the thin membrane is not easily damaged and has an appropriate strength, but since the holding body such as a gauze absorbs water and expands, the problem that the membrane bends remains.

The present invention was made in view of the above circumstances, and provides a semipermeable membrane having a moderate strength which is difficult to bend and easy to handle. In addition, the present invention provides a cell-culturing device which not only has an excellent cell protection performance but is also easy to handle, which is able to carry out cell culturing for a long period of time, and with which it is possible to measure the number of cells.

## Solution to Problem

As a result of intensive research to solve the above problems, the present inventors found that, by producing a cell-culturing device provided with a semipermeable membrane having a lattice structure and a low water absorption property and injecting and culturing cells in the cell-culturing device, it is possible to obtain an easy-to-handle and highly functional tissue-type chip which is able to be held with tweezers without bending the membrane, thus completing the present invention.

That is, the present invention includes the following aspects.

The semipermeable membrane according to a first aspect of the present invention includes a holding body with a low water absorption property having a lattice structure and having a semipermeable property in a liquid phase.

In the semipermeable membrane of the first aspect described above, the lattice structure of the holding body may function as a scale of micrometer units.

In the semipermeable membrane of the first aspect described above, the holding body may be formed of polyester or polystyrene.

The semipermeable membrane of the first aspect described above may further include a material having biocompatibility.

In the semipermeable membrane of the first aspect described above, the material having biocompatibility may be a component derived from an extracellular matrix available for gelation.

In the semipermeable membrane of the first aspect described above, the component derived from the extracellular matrix available for gelation may be native collagen or atelocollagen.

A cell-culturing device according to a second aspect of the present invention includes the semipermeable membrane according to the first aspect described above in at least a part thereof.

The cell-culturing device according to the second aspect described above may further have liquid-tightness in a gas phase.

The cell-culturing device according to the second aspect described above into which cells suspended in a culture medium may be able to be injected and in which an internal volume may be 10 mL or less.

In the cell-culturing device according to the second aspect described above, the whole device is formed of the semipermeable membrane.

A tissue-type chip according to a third aspect of the present invention includes the cell-culturing device according to the second aspect described above, including one type of cells.

In the tissue-type chip according to the third aspect described above, a density of the cells may be 2.0 $\times$ 10$^3$ cells/mL or more and 1.0 $\times$ 10$^9$ cells/mL or less.

An organ-type chip according to a fourth aspect of the present invention includes the cell-culturing device according to the second aspect, including at least two types of cells.

In the organ-type chip according to the fourth aspect described above, a density of the cells may be 2.0 $\times$ 10$^3$ cells/mL or more and 1.0 $\times$ 10$^9$ cells/mL or less.

A kit according to a fifth aspect of the present invention, which is a kit for providing a multicellular structure, includes an openable and closable sealed container including the tissue-type chip according to the third aspect described above or the organ-type chip according to the fourth aspect described above, and a culture medium.

An organ-type chip system according to a sixth aspect of the present invention includes at least two of the tissue-type chips according to the third aspect described above or the organ-type chips according to the fourth aspect described above in which the tissue-type chips or the organ-type chips are connected while maintaining a sealing property.

A cell-culturing method according to a seventh aspect of the present invention is a method using the cell-culturing device according to the second aspect described above.

A method for measuring a number of cells according to an eighth aspect of the present invention is a method using the cell-culturing device according to the second aspect described above.

Advantageous Effects of Invention

The semipermeable membrane of the above aspects has a moderate strength which is difficult to bend and easy to handle. In addition, the cell-culturing device of the aspects described above not only has an excellent cell protection performance but is also easy to handle, is able to carry out cell culturing for a long period of time, and makes it possible to measure the number of cells.

Brief Description of Drawings

FIG. 1 is a plan view schematically showing a semipermeable membrane according to a first embodiment of the present invention.

FIG. 2 is a perspective view schematically showing a cell-culturing device according to the first embodiment of the present invention.

FIG. 3 is a perspective view schematically showing a cell-culturing device according to a second embodiment of the present invention.

FIG. 4 is a perspective view schematically showing a cell-culturing device according to a third embodiment of the present invention.

FIG. 5A is a perspective view schematically showing a cell-culturing device (the inside and the outside of the device communicate via an injection hole) according to a fourth embodiment of the present invention.

FIG. 5B is a perspective view schematically showing the cell-culturing device (the inside and the outside of the device do not communicate) according to the fourth embodiment of the present invention.

FIG. 6A is a cross-sectional view schematically showing a cell-culturing device (the inside and the outside of the device communicate via an injection hole) according to a fifth embodiment of the present invention.

FIG. 6B is a cross-sectional view schematically showing the cell-culturing device (the inside and the outside of the device do not communicate) according to the fifth embodiment of the present invention.

FIG. 7 is a perspective view schematically showing a cell-culturing device according to a sixth embodiment of the present invention.

FIG. 8A is a perspective view schematically showing an organ-type chip system according to the first embodiment of the present invention.

FIG. 8B is a perspective view schematically showing an organ-type chip system according to the second embodiment of the present invention.

FIG. 8C is a perspective view schematically showing an organ-type chip system according to the third embodiment of the present invention.

FIG. 8D is a perspective view schematically showing an organ-type chip system according to the fourth embodiment of the present invention.

FIG. 9 is an image showing a semipermeable membrane produced in Production Example 1.

FIG. 10A is an image showing a state in Example 1 in which PBS is injected into a cell-culturing device provided with a semipermeable membrane in which a holding body is embedded and pinched with tweezers.

FIG. 10B is an image showing a state in Example 1 in which PBS is injected into a cell-culturing device provided with a semipermeable membrane in which the holding body is embedded and left to stand still vertically.

FIG. 11A is an image showing a state in Example 1 in which PBS is injected into a cell-culturing device provided with a semipermeable membrane not including a holding body and pinched with tweezers.

FIG. 11B is an image showing a state in Example 1 in which PBS is injected into a cell-culturing device provided with a semipermeable membrane not including a holding body and left to stand still vertically.

FIG. 12A is an image showing a state of HepG2 cells on the first day of culturing in Test Example 1.

FIG. 12B is an image showing a state of HepG2 cells on the second day of culturing in Test Example 1.

FIG. 12C is an image showing a state of HepG2 cells on the fourth day of culturing in Test Example 1.

FIG. 12D is an image showing a state of HepG2 cells on the seventh day of culturing in Test Example 1.

FIG. 12E is an image showing a state of HepG2 cells on the tenth day of culturing in Test Example 1.

FIG. 12F is an image showing a state of HepG2 cells on the fourteenth day of culturing in Test Example 1.

Description of Embodiments

A more detailed description will be given below of the present invention with reference to embodiments; however, the present invention is not at all limited to the following embodiments.

"Semipermeable Membrane"

In one embodiment, the present invention provides a semipermeable membrane including a holding body with a low water absorption property having a lattice structure and having a semipermeable property in a liquid phase.

The semipermeable membrane of the present embodiment has a moderate strength which is difficult to bend and easy to handle. Therefore, in the cell-culturing device provided with the semipermeable membrane of the present embodiment, it is possible to hold the membrane without bending and without damage even when pinching with tweezers, and handling is easy.

<Structure>

[First Embodiment]

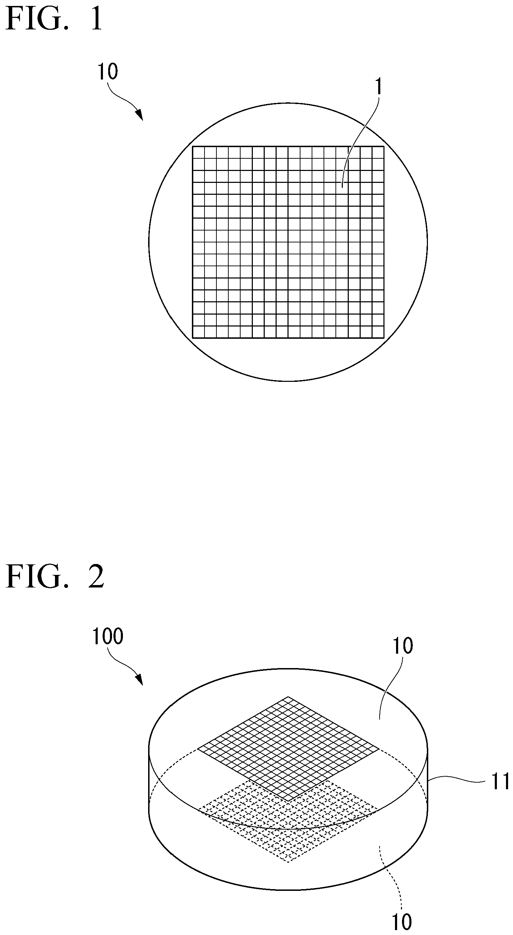

FIG. 1 is a plan view schematically showing a semipermeable membrane according to a first embodiment of the present invention. A semipermeable membrane 10 shown here includes a holding body 1 with a low water absorption property and having a lattice structure. The holding body 1 may be adhered to at least a part of the surface of the semipermeable membrane or may be in a state where at least a part thereof is embedded in the semipermeable membrane.

The semipermeable membrane 10 has a semipermeable property in a liquid phase. In the present specification, "semipermeable" means a property which only allows the passage of molecules or ions having a certain molecular weight or less, and a "semipermeable membrane" is a membrane having such a property. In the cell-culturing device provided with the semipermeable membrane of the present embodiment, for example, in a case where the cell-culturing device including cells is immersed in a container including a culture medium, the semipermeable membrane does not allow cells to pass to the outside of the cell-culturing device, while the nutrients dissolved in the culture medium are allowed to pass to the inside of the cell-culturing device and cell products including waste matter dissolved in the culture medium inside the cell-culturing device are allowed to pass to the outside of the cell-culturing device. Therefore, it is possible to use the cell-culturing device of the present embodiment for cell culturing for a long period of time. More specifically, it is possible to set the semipermeable membrane of the present embodiment, for example, to allow a polymer compound having a molecular weight of about 1,000,000 or less to pass therethrough, for example, to allow a molecular compound having a molecular weight of about 200,000 or less to pass therethrough.

In FIG. 1, the semipermeable membrane 10 is shown with a circular shape, but may have other shapes and examples thereof include a polygon (including a regular polygon or the like), an ellipse, a fan, and the like, without being limited thereto.

In addition, in FIG. 1, the holding body 1 is shown as a square, but may have other shapes and examples thereof include a polygon (including a regular polygon or the like), an ellipse, a fan, and the like, without being limited thereto.

<Constituent Materials>

[Holding Body]

The holding body in the present embodiment preferably has a lattice and a low water absorption property.

In the present specification, "lattice" means a state in which a plurality of vertical lines and horizontal lines intersect perpendicularly with each other. The holding body in the present embodiment preferably has a portion in which the vertical lines and the horizontal lines are each arranged at equal intervals in units of micrometers. That is, the holding body in the present embodiment preferably functions as a scale of micrometer units. Due to this, the cell-culturing device provided with the semipermeable membrane of the present embodiment is able to be used as a substitute for a hemocytometer in a case of including cells, and the number of cells included in the cell-culturing device is easily measurable.

The intervals (that is, the mesh openings) of each of the vertical lines and the horizontal lines forming the lattice are preferably 100 μm or more and 500 μm or less, and more preferably 200 μm or more and 300 μm or less.

In addition, it is possible to set the line diameter of the vertical lines and the horizontal lines forming the lattice to be, for example, 0.1 μm or more and 100 μm or less, and, for example, 1 μm or more and 80 μm or less.

In addition, in the present specification, "low water absorption property" means that the water absorption rate measured by the Japanese Industrial Standard (JIS K 7209) is low. Specifically, the water absorption rate is preferably less than 1%.

As the material of the holding body in the present embodiment, it is possible to use a material which is plastic, which is able to form a lattice by processing fibers (threads) or a film, which has low water absorption property and moderate hardness, and which has low cytotoxicity. Examples of the plastic include polyvinyl chloride, styrene copolymer, polyacrylate (acrylic resin), polycarbonate, polyester (particularly polyethylene terephthalate), urea resin, phenol resin, melamine resin, polyacetal, polyethylene, polypropylene, polytetrafluoride ethylene, polyfluoroethylene, polyvinylidene chloride, polystyrene, and the like, without being limited thereto.

More specific examples of the polyacrylate (acrylic resin) include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), and the like.

Among these, the material of the holding body in the present embodiment is preferably polyester or polystyrene due to the production cost and the fact that the semipermeable membrane of the present embodiment is usually used for handling cells, and polyethylene terephthalate or polystyrene is more preferable.

As the method of producing the holding body, it is possible to carry out the producing by using a known method of producing a mesh using fibers (threads) or a film of a plastic resin.

More specifically, as the method of producing a holding body using fibers (threads), the producing is carried out by first weaving fibers (threads) having a desired line diameter formed of the material described above into a lattice using a machine or the like. At this time, the intersection points of the vertical fibers (thread) and the horizontal fibers (thread) may be fused by applying heat, pressure, or the like. Fusing the intersection points makes it possible to obtain a smooth holding body with no level differences.

In addition, as a method of producing a holding body using a film, more specifically, it is possible to carry out the producing by first cutting pores in a film having a desired film thickness formed of the material described above in a lattice using a machine or the like. At this time, the porous shape may be uniform and function as a scale of micrometer units, and examples thereof include a polygon (including a square), a circle, an ellipse, and the like, without being limited thereto.

[Others]

In the semipermeable membrane of the present embodiment, as a constituent material other than the holding body, it is possible to use a material having no cytotoxicity, which may be a natural polymer compound or a synthetic polymer compound. In addition, as a material having the above properties, a material having biocompatibility is preferable. In the present specification, "biocompatibility" means an evaluation criterion indicating compatibility between living tissue and the material, and "having biocompatibility" means a state in which the material itself has no toxicity, components derived from microorganisms such as endotoxins are not present, the living tissue is not physically stimulated, and rejection does not occur even when proteins, cells, or the like forming the living tissue interact with each other.

Examples of natural polymer compounds having biocompatibility include components derived from an extracellular matrix available for gelation, polysaccharides (for example, alginate, cellulose, dextran, pullulane, polyhyaluronic acid, derivatives thereof, and the like), chitin, poly(3-hydroxyalkanoate) (in particular, poly(β-hydroxybutyrate), poly(3-hydroxyoctanoate)), poly(3-hydroxy fatty acid), fibrin, agar, agarose, and the like, without being limited thereto.

The cellulose also includes cellulose modified by synthesis, and examples thereof include cellulose derivatives (for example, alkyl cellulose, hydroxyalkyl cellulose, cellulose ether, cellulose ester, nitrocellulose, chitosan, or the like) and the like. More specific examples of cellulose derivatives include methylcellulose, ethylcellulose, hydroxypropylcellulose, hydroxypropylmethylcellulose, hydroxybutylmethylcellulose, cellulose acetate, cellulose propionate, cellulose acetate butyrate, cellulose acetate phthalate, carboxymethylcellulose, cellulose triacetate, cellulose sulfate sodium salt, and the like.

Among these, the natural polymer compound is preferably a component derived from an extracellular matrix available for gelation, fibrin, agar, or agarose since these have excellent water retention.

Examples of components derived from an extracellular matrix available for gelation include collagen (type I, type II, type III, type V, type XI, or the like), a reconstituted basement membrane component (trade name: Matrigel) derived from mouse EHS tumor extract (including type IV collagen, laminin, heparan sulfate proteoglycan, or the like), glycosaminoglycan, hyaluronic acid, proteoglycans, gelatin, and the like, without being limited thereto. It is possible to produce semipermeable membranes by selecting components suitable for gelation such as salts, the concentrations and pH thereof, and the like. In addition, combining the raw materials makes it possible to obtain a semipermeable membrane imitating various tissues in living bodies.

Examples of synthetic polymer compounds having biocompatibility include polyphosphazene, poly(vinyl alcohol), polyamide (such as nylon), polyester amide, poly(amino acid), polyanhydride, polysulfone, polycarbonate, polyacrylate (acrylic resin), polyalkylene (for example, polyethylene and the like), polyacrylamide, polyalkylene glycol (for example, polyethylene glycol and the like), polyalkylene oxide (for example, polyethylene oxide and the like), polyalkylene terephthalate (for example, polyethylene terephthalate and the like), polyorthoester, polyvinyl ether, polyvinyl ester, polyvinyl halide, polyvinyl pyrrolidone, polyester, polysiloxane, polyurethane, polyhydroxy acid (for example, polylactide, polyglycolide, and the like), poly(hydroxybutyric acid), poly(hydroxyvaleric acid), poly[lactide-co-($\varepsilon$-caprolactone)], poly[glycolide-co-($\varepsilon$-caprolactone)], or the like), poly(hydroxyalkanoate), copolymers thereof, and the like, without being limited thereto.

Among these, as the synthetic polymer compound, polyhydroxy acids (for example, polylactide, polyglycolide, and the like), polyethylene terephthalate, poly(hydroxybutyric acid), poly(hydroxyvaleric acid), poly[lactide-co-($\varepsilon$-caprolactone)], poly[glycolide-co-($\varepsilon$-caprolactone)], or the like), poly(hydroxyalkanoate), polyorthoester, or copolymers thereof are preferable.

In the semipermeable membrane in the present embodiment, the constituent material other than the holding body may be formed of one type of the materials exemplified above, or may be formed of two or more types thereof. In addition, the material of the semipermeable membrane in the present embodiment may be formed of any of natural polymer compounds or synthetic polymer compounds, or may be formed of both natural polymer compounds and synthetic polymer compounds.

In addition, in a case where the semipermeable membrane is formed of two or more types of the materials exemplified above, the semipermeable membrane may be formed of a mixture of the materials exemplified above. Alternatively, the semipermeable membrane may be formed by laminating two or more layers of semipermeable membranes formed of one type of material, and the materials forming each semipermeable membrane may be formed of different membranes.

Among these, in the semipermeable membrane of the present embodiment, as a constituent material other than the holding body, a natural polymer compound is preferable, a component derived from an extracellular matrix available for gelation is more preferable, and collagen is even more preferable. In addition, among collagens, examples of more preferable raw materials include native collagen and atelocollagen.

In a case where the material of the semipermeable membrane in the present embodiment is a component derived from an extracellular matrix, the component derived from an extracellular matrix is preferably contained in an amount of 0.1 mg or more and 10.0 mg or less per 1 $cm^2$ unit area of the semipermeable membrane, and more preferably contained in an amount of 0.5 mg or more and 5.0 mg or less. In particular, in a case where the component derived from the extracellular matrix is collagen, collagen is preferably contained in an amount of 0.2 mg or more and 10.0 mg or less per 1 $cm^2$ of the unit area of the semipermeable membrane, and more preferably contained in an amount of 0.25 mg or more and 5.0 mg or less per 1 $cm^2$.

The content of the component derived from the extracellular matrix (in particular, collagen) in the semipermeable membrane being in the ranges described above makes it possible to have strength such that it is possible to inject and culture the cells in the cell-culturing device.

The "weight per unit area of 1 $cm^2$ of the membrane" refers to the weight of the component contained per 1 $cm^2$ of the material piece, with the thickness of the membrane being arbitrary.

The thickness of the semipermeable membrane in the present embodiment is not particularly limited; however, the thickness is preferably 1 $\mu$m or more and 1000 $\mu$m or less, more preferably 1 $\mu$m or more and 500 $\mu$m or less, even more preferably 5 $\mu$m or more and 300 $\mu$m or less, and particularly preferably 10 $\mu$m or more and 200 $\mu$m or less. The thickness of the semipermeable membrane being within the above range makes it possible to have strength such that it is possible to inject and culture the cells in the cell-culturing device.

Here, the "thickness of the semipermeable membrane" means the thickness of the semipermeable membrane as a whole, for example, the thickness of the semipermeable membrane formed of a plurality of layers means the total of all the layers forming the semipermeable membrane.

In addition, the semipermeable membrane in the present embodiment does not break during use and is excellent in practical use.

<Method of Producing Semipermeable Membrane>

1. Method of Producing a Semipermeable Membrane using Synthetic Polymer Compound having Biocompatibility

For example, in a case where the constituent material of the semipermeable membrane is a synthetic polymer compound having biocompatibility, it is possible to produce the semipermeable membrane using a known method (for example, refer to Japanese Unexamined Patent Application, First Publication No. 2001-149763 or the like).

More specifically, as a method for producing a semipermeable membrane using a synthetic polymer compound having biocompatibility, first, a membrane-forming stock solution in which a synthetic polymer compound is dissolved in an organic solvent is prepared.

As the organic solvent, it is possible to use any solvent for the synthetic polymer compound, and examples thereof include tetrahydrofuran, dioxane, dimethylformamide, dimethylacetamide, N-methyl-2-pyrrolidone, and the like, without being limited thereto.

It is possible to appropriately adjust the mixing ratio of the synthetic polymer compound and the organic solvent according to the types of the synthetic polymer compound and the organic solvent to be used, for example, it is possible to set the synthetic polymer compound to be 15% by weight and the organic solvent to be 85% by weight.

In addition, it is usually possible to set the temperature of the organic solvent at the time of dissolution to be 30°C or more and 100°C or less, and preferably 50°C or more and 80°C or less.

Next, using, for example, a method of discharging from a nozzle, the prepared membrane-forming stock solution is coagulated in a coagulating liquid and a semipermeable membrane with a predetermined shape is produced. At this time, it is possible to produce the semipermeable membrane arranging the holding body in the coagulating liquid and discharging the membrane-forming stock solution thoroughly so as to include the holding body.

As the coagulating liquid, a mixed solution of an organic solvent and water is preferably used. As the organic solvent used for the coagulating liquid, it is possible to use the same organic solvents as those exemplified as the organic solvent used for dissolving the synthetic polymer compound. The organic solvent used for the coagulating liquid may be of the same type as the organic solvent used for dissolving the synthetic polymer compound or may be of a different type.

In addition, it is possible to set the ratio of water in the coagulating liquid to be, for example, 30% by weight or more and 80% by weight or less.

Furthermore, for the purpose of adjusting the coagulation rate, alcohols such as methanol, ethanol, isopropanol, and glycerin, and glycols such as ethylene glycol and propylene glycol may be added to the coagulating liquid.

Alternatively, the semipermeable membrane may be produced by sandwiching the holding body using two films solidified into a predetermined shape and adhering using an adhesive and drying.

As the adhesive, it is possible to use an adhesive having no cytotoxicity, and the adhesive may be an adhesive of a synthetic compound or an adhesive of a natural compound.

Examples of the adhesive of the synthetic compound include a urethane adhesive, a cyanoacrylate adhesive, polymethyl methacrylate (PMMA), a calcium phosphate adhesive, a resin type cement, and the like.

Examples of the adhesive of the natural compound include fibrin glue, gelatin glue, and the like.

It is possible to use the obtained semipermeable membrane by washing with distilled water or the like and further sterilizing by ultraviolet ray irradiation or the like.

2. Method for Producing Semipermeable Membrane using Hydrogel

In addition, for example, in a case where the constituent material of the semipermeable membrane is a hydrogel, it is possible to produce the semipermeable membrane using a known method (for example, refer to Japanese Unexamined Patent Publication No. 8-228768, PCT International Publication No. WO 2012/026531, Japanese Unexamined Patent Application, First Publication No. 2012-115262, Japanese Unexamined Patent Application, First Publication No. 2015-35978, and the like).

In the present specification, the term "hydrogel" refers to a substance in which the polymer compound has a network structure due to chemical bonding and which has a large amount of water in the network thereof, more specifically, the hydrogel means a substance obtained by introducing cross-linking into an artificial material of a natural polymer compound or synthetic polymer compound to cause gelation.

Examples of hydrogels include natural polymer compounds such as the above-described component derived from the extracellular matrix available for gelation, fibrin, agar, agarose, and cellulose, and synthetic polymer compounds such as polyacrylamide, polyvinyl alcohol, polyethylene oxide, and poly(II-hydroxyethylmethacrylate)/polycaprolactone, and the like.

More specifically, as a method for producing a semipermeable membrane using a hydrogel, first, a hydrogel which is in a state of being not completely gelled (may be referred to below as "sol") is arranged in a mold in which a holding body is arranged in advance and gelation is induced.

In a case where the sol is a collagen sol, it is possible to use a collagen sol which is prepared using physiological saline, phosphate-buffered saline (PBS), Hank's Balanced Salt Solution (HBSS), a basic culture medium, a serum-free culture medium, a serum-containing culture medium, or the like to have an optimal salt concentration. In addition, the pH of the solution at the time of collagen gelation may be, for example, 6 or more and 8 or less.

In addition, the collagen sol may be prepared at approximately 4°C, for example. Thereafter, it is possible for the preserved temperature during gelation to be lower than the denaturation temperature of collagen depending on the animal species of collagen to be used, and, generally, it is possible to perform gelation in several minutes to several hours by maintaining a preserved temperature of 20°C or more and 37°C or less.

In addition, the concentration of the collagen sol for producing the semipermeable membrane is preferably 0.1% or more and 1.0% or less, and more preferably 0.2% or more and 0.6% or less. When the concentration of the collagen sol is the above lower limit value or more, the gelation is not too weak, and when the concentration of the collagen sol is the above upper limit value or less, it is possible to obtain a semipermeable membrane formed of uniform collagen gel.

Furthermore, the obtained hydrogel may be dried to obtain a hydrogel dried body. Drying the hydrogel makes it possible to completely remove the free water in the hydrogel and to further proceed with the partial removal of bonding water.

The longer the period of this vitrification step (the step of proceeding with the removal of a portion of bonding water after completely removing free water in the hydrogel), the more it is possible to obtain a hydrogel after vitrification with excellent transparency and strength when rehydrated, that is, Vitrigel (registered trademark). After a short period of vitrification, the Vitrigel (registered trademark) obtained by rehydration is washed with PBS or the like and it is also possible to carry out the vitrification again as necessary.

In the present specification, "Vitrigel (registered trademark)" refers to a gel in a stable state obtained by vitrification and subsequent rehydration of a hydrogel in the related art and it was the present inventor who named "Vitrigel (registered trademark)".

As a drying method, for example, it is possible to use various methods such as air drying, drying in a sealed container (circulating air in a container, and constantly supplying dry air), drying in an environment in which silica gel is placed, and the like. For example, examples of methods of air drying include methods such as drying for 2 days in an incubator kept sterile at 10°C and 40% humidity, or drying in a clean bench in a sterile state for 24 hours at room temperature.

Furthermore, by rehydrating the dried body of the obtained Vitrigel (registered trademark) again with PBS, a culture medium to be used, or the like, the dried body may be used again as the Vitrigel (registered trademark).

In addition, in the present specification, when describing in detail the steps of producing a semipermeable membrane formed of a hydrogel, the hydrogel dried body immediately after the vitrification step and not subjected to a rehydration step is simply referred to as a "hydrogel dried body". Then, the gel obtained through the rehydration step after the vitrification step is expressed distinctly as "Vitrigel (registered trademark)" and the dried body obtained by vitrifying Vitrigel (registered trademark) is referred to as "Vitrigel (registered trademark) dried body". In addition, a product obtained by subjecting a Vitrigel (registered trademark) dried body to a step of ultraviolet ray irradiation is referred to as a "Vitrigel (registered trademark) material dried body subjected to an ultraviolet ray irradiation treatment" and a gel obtained by carrying out a step of rehydrating the "Vitrigel (registered trademark) material dried body" is referred to as a "Vitrigel (registered trademark) material". Accordingly, "Vitrigel (registered trademark)" and "Vitrigel (registered trademark) material" are hydrates.

That is, the obtained Vitrigel (registered trademark) may be re-dried to carry out re-vitrification to obtain a Vitrigel (registered trademark) dried body.

Examples of the drying method include the same methods as described above.

In addition, the obtained Vitrigel (registered trademark) dried body may be irradiated with ultraviolet rays to obtain the "Vitrigel (registered trademark) material dried body which is the Vitrigel (registered trademark) dried body subjected to an ultraviolet ray irradiation treatment".

For ultraviolet ray irradiation, it is possible to use a known ultraviolet ray irradiation apparatus.

The total irradiation amount per unit area of ultraviolet ray irradiation energy to Vitrigel (registered trademark) dried body is preferably 0.1 mJ/cm$^2$ or more and 6000 mJ/cm$^2$ or less, more preferably 10 mJ/cm$^2$ or more and 4000 mJ/cm$^2$ or less, and even more preferably 100 mJ/cm$^2$ or more and 3000 mJ/cm$^2$ or less. When the total irradiation amount is in the above range, it is possible for the transparency and strength of Vitrigel (registered trademark) material obtained in the subsequent rehydration step to be particularly preferable.

In addition, the irradiation of the Vitrigel (registered trademark) dried body with ultraviolet rays may be repeated a plurality of times. In a case of repeating the irradiation of the Vitrigel (registered trademark) dried body with ultraviolet rays, it is preferable that, after the first irradiation of the Vitrigel (registered trademark) dried body with the ultraviolet rays, the steps of rehydration and re-vitrification of the Vitrigel (registered trademark) material dried body in which the Vitrigel (registered trademark) dried body is subjected to ultraviolet ray irradiation treatment be performed and then the Vitrigel (registered trademark) material dried body after second and subsequent re-vitrification be irradiated with ultraviolet rays.

When the total ultraviolet ray irradiation amount per unit area is the same, the Vitrigel (registered trademark) dried body is repeatedly irradiated with ultraviolet rays while being divided a plurality of times, such that it is possible to further increase the transparency and strength of the Vitrigel (registered trademark) material obtained in the following rehydration step. In addition, the larger the number of divisions, the better. For example, when the total irradiation amount per unit area of the ultraviolet ray irradiation on the Vitrigel (registered trademark) dried body is in the range of 1000 mJ/cm$^2$ or more and 4000 mJ/cm$^2$ or less, the number of times of irradiation in the above range is preferably 2 times or more and 10 times or less, and more preferably 2 times or more and 6 times or less.

In addition, in a case of repeating the irradiation of the Vitrigel (registered trademark) dried body with ultraviolet rays, the irradiation is carried out after dividing the irradiation site of the ultraviolet rays into one side of the Vitrigel (registered trademark) dried body and the other side (the upper side and the lower side), and the total irradiation amount may be the total ultraviolet irradiation amount per unit area on the Vitrigel (registered trademark) dried body.

It is considered that the increase in the strength and transparency of the obtained Vitrigel (registered trademark) material in the subsequent rehydration step by irradiating the Vitrigel (registered trademark) dried body with ultraviolet rays is because the polymer compounds in the Vitrigel (registered trademark) material are cross-linked by the ultraviolet rays. In other words, it is considered that, through this operation, it is possible to maintain high transparency and strength in the Vitrigel (registered trademark) material.

Furthermore, the Vitrigel (registered trademark) material dried body in which the obtained Vitrigel (registered trademark) material is subjected to ultraviolet irradiation treatment may be rehydrated with PBS, the culture medium to be used, or the like to obtain the Vitrigel (registered trademark) material.

Furthermore, a Vitrigel (registered trademark) material dried body may be obtained by drying the obtained Vitrigel (registered trademark) material to carry out re- vitrification.

Examples of the drying method include the same methods as described above.

Alternatively, using the above-described production method, the semipermeable membrane may be produced using two membranes formed of hydrogel, a hydrogel dried body, Vitrigel (registered trademark), Vitrigel (registered trademark) dried body, Vitrigel material, or Vitrigel (registered trademark) material dried body of approximately half the normal thickness to sandwich the holding body and carrying out adhesion using an adhesive or a sol and carrying out drying.

As the adhesive, it is possible to use adhesives which are not cytotoxic, and examples thereof include the same adhesives as exemplified in the above "1. Method of Producing a Semipermeable Membrane using Synthetic Polymer Compound having Biocompatibility".

"Cell-culturing device"

In one embodiment, the present invention provides a cell-culturing device provided with the semipermeable membrane described above in at least a part thereof.

In the cell-culturing device of the present embodiment, it is possible to hold the membrane without bending and without damage even when pinching the semipermeable membrane with tweezers, or the like.

In addition, in the related art, there is a time restriction in that, after a multicellular structure is produced as a culture model, the multicellular structure has to be used within 1 to 3 days. In contrast, the cell-culturing device of the present embodiment is able to culture cells for a long period of approximately 3 to 30 days, and is not subject to a time restriction.

Furthermore, since the cell-culturing device of the present embodiment includes the holding body inside or on the surface of the semipermeable membrane (the outside of the top surface or the bottom surface of the cell-culturing device), the cell-culturing device is able to be used as a substitute for a hemocytometer, and the number of cells included in the cell-culturing device is easily measurable.

The cell-culturing device of the present embodiment is provided with the semipermeable membrane described above in at least a part thereof. Therefore, for example, in a case where the cell-culturing device of the present embodiment including the cells is immersed in a container including the culture medium, the semipermeable membrane does not allow cells to pass to the outside of the cell-culturing device, while the nutrients dissolved in the culture medium are allowed to pass to the inside of the cell-culturing device and cell products including waste matter dissolved in the culture medium inside the cell-culturing device are allowed to pass to the outside of the cell-culturing device. Therefore, it is possible to use the cell-culturing device of the present embodiment to culture cells for a long period.

The cell-culturing device of the present embodiment preferably further has liquid-tightness in a gas phase.

In the present specification, "liquid-tightness" means a state in which liquid does not leak. In the cell-culturing device according to the present embodiment, for example, in a case where a liquid such as a culture medium is included inside, it is possible to preserve the inside without liquid leaking from any surface in the gas phase. On the other hand, since it is possible for gas to pass therethrough, in a case where a liquid is included inside, the liquid inside evaporates over time. Therefore, the cell-culturing device of the present embodiment is able to preserve a state in which cells are sealed inside.

In the present specification, "multicellular structure" means a three-dimensional structure formed of monolayer cells or multi-layered cells in which a plurality of cells form cell-substratum bonds and cell-cell bonds.

The multicellular structure in the present embodiment is formed of one or more types of functional cells and a substratum which has the role of a scaffold. That is, in the multicellular structure in the present embodiment, a plurality of functional cells interact with a substratum to construct a form which is more similar to tissues or organs in a living body. Accordingly, capillary network-like structures such as at least blood vessels and/or bile ducts may be three-dimensionally constructed in the multicellular structure. Such capillary network-like structures may be formed only inside the multicellular structure, or may be formed such that at least a portion thereof is exposed on the surface or the bottom surface of the multicellular structure.

<Structure>

[First Embodiment]

FIG. 2 is a perspective view schematically showing a cell-culturing device according to a first embodiment of the present invention. A cell-culturing device 100 shown here is provided with semipermeable membranes 10 on the top and bottom surfaces and has the shape of a cylinder sealed on the side surface by a member 11. In FIG. 2, a cell-culturing device is exemplified in which the semipermeable membrane is provided on the top surface and the bottom surface, but the semipermeable membrane may be provided on a part of the top surface, the bottom surface, the side surface, or the like, and the entirety of the top surface, the bottom surface, the side surface, and the like may be formed of a semipermeable membrane. Among these, in a case where the cell-culturing device of the present embodiment is used as a culture model, a semipermeable membrane is preferably provided on the top surface and the bottom surface.

In FIG. 2, the cell-culturing device is shown to have a cylindrical shape, but the cell-culturing device of the present embodiment may be any shape as long as it is possible for cells to be stored therein and oxygen and nutrients uniformly dissolved in the culture medium are distributed to the cells, and the inside of the device may be filled with the culture medium, or a gas portion may be left without being filled with the culture medium. Examples of the shape of the cell-culturing device of the present embodiment include a cylindrical shape (for example, a ring-shaped cylinder, a hollow fiber-like cylinder or the like), a circular cone, a circular truncated cone, a pyramid, a truncated pyramid, a sphere, a polyhedron (for example, a tetrahedron, a pentahedron, a hexahedron (including cubes), an octahedron, a dodecahedron, an icosahedron, an icositetrahedron, a Kepler-Poinsot polyhedron, or the like), and the like, without being limited thereto.

In a case where the shape of the cell-culturing device is a ring-shaped cylinder as shown in FIG. 2, the inner diameter of the cell-culturing device is preferably 1 mm or more and 60 mm or less, more preferably 3 mm or more and 35 mm or less, and even more preferably 5 mm or more and 30 mm or less.

In addition, the outer diameter of the cell-culturing device is preferably 3 mm or more and 68 mm or less, more preferably 5 mm or more and 43 mm or less, and even more preferably 7 mm or more and 35 mm or less.

In addition, the thickness of the cell-culturing device (ring-shaped cylinder height) is 5 $\mu$m or more, preferably 50 $\mu$m or more and 15 mm or less, more preferably 100 $\mu$m or more and 10 mm or less, and even more preferably 500 $\mu$m or more and 2 mm or less.

In the present specification, the "thickness of the cell-culturing device (ring-shaped cylinder height)" means the distance from the outer edge of the top surface of the cell-culturing device to the outer edge of the bottom surface of it.

Although the top surface and the bottom surface are shown to be flat in FIG. 2, the top surface and the bottom surface may have a concave structure or a convex structure. In particular, when the top surface and the bottom surface have a concave structure, the center portion of the concave portion on the inner side of the top surface (the most concave portion on the inner side of the top surface) and the center portion of the concave portion on the inner side of the bottom surface (the most concave portion on the inner side of the bottom surface) are preferably maintained at a certain distance (for example, 5 $\mu$m or more) without coming into contact. Due to this, the thickness of the cell-culturing device (ring-shaped cylinder height), that is, the distance from the outer edge of the top surface of the cell-culturing device to the outer edge of the bottom surface of it is longer than the distance from the center portion of the concave portion on the outside of the top surface (the most concave portion on the outside of the top surface) to the center portion of the concave portion on the outside of the bottom surface (the most concave portion on the outside of the bottom surface), and, for example, in a case where a medicine is added from the top surface, it is possible to maintain the directionality of the added medicine. Furthermore, since the top surface and the bottom surface have a concave structure, it is possible to newly seed and culture cells outside the top surface and the bottom surface.

In addition, in FIG. 2, the top surface, the bottom surface, and members of the side surface are joined perpendicularly, but at least one edge portion of the top surface and the bottom surface may be joined to the members of the side surface while drawing a linear, convex curved, concave curved, or substantially S-shaped curve slope. In particular, in a case where the edge of the bottom surface is joined to the member on the side surface while drawing the inclination of the slope described above, when lifting the top and bottom surfaces of the cell-culturing device of the present embodiment by using tweezers or the like, the tweezers or the like enter into the inclined portion, and it is possible to carry out the lifting easily.

The internal volume of the cell-culturing device of the present embodiment is able to inject cells suspended in a culture medium and to achieve a small scale in which it is possible to construct a multicellular structure to be used in an *in vitro* test system such as a test for assaying cell activity, preferably 10 mL or less, more preferably 10 μL or more and 5 mL or less, even more preferably 15 μL or more and 2 mL or less, and particularly preferably 20 μL or more and 1 mL or less. When the internal volume is the upper limit value or less described above, oxygen and nutrients in the culture medium are sufficiently supplied, and it is possible to efficiently culture cells over a long period of time. In addition, when the internal volume is the lower limit value or more described above, it is possible to obtain a sufficient number of cells and cell density for use in an *in vitro* test system.

[Second Embodiment]

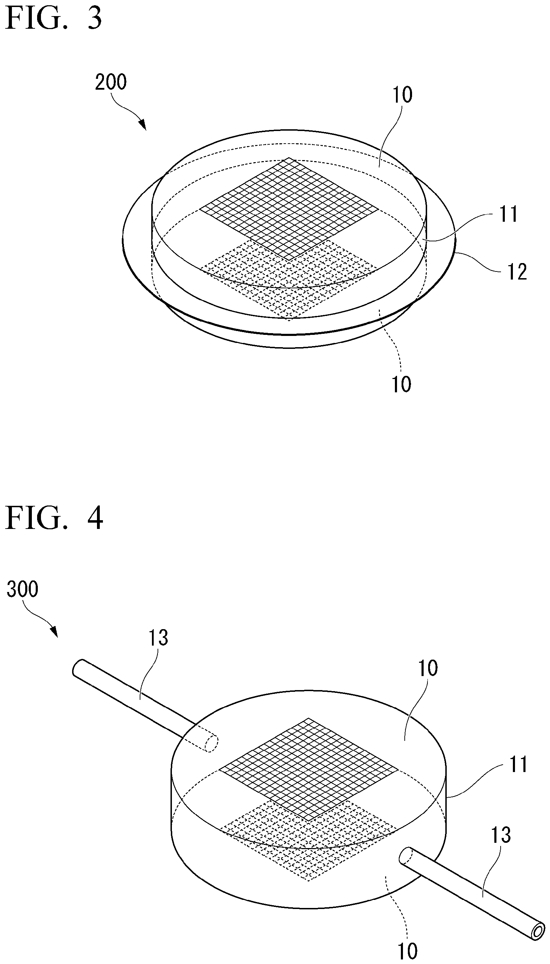

FIG. 3 is a perspective view schematically showing a cell-culturing device according to a second embodiment of the present invention.

In the diagrams following FIG. 3, the same reference numerals are given to the same constituent elements as in the cases of the already explained diagrams and detailed descriptions thereof will be omitted.

A cell-culturing device 200 shown here is the same as the cell-culturing device 100 shown in FIG. 2 except for being provided with a support 12. That is, the cell-culturing device 200 is provided with the semipermeable membrane 10 on the top surface and the bottom surface, has the shape of a cylinder sealed on the side surface by the member 11 and is provided with the support 12 on the outer surface.

The cell-culturing device 200 having the support 12 makes it possible for the cell-culturing device 200 to culture the cells in a gas phase by, for example, fixing the cell-culturing device 200 in which the cells are included in a container one size larger. In addition, the cell-culturing device 200 having the support 12 means that, for example, the cell-culturing device 200 in which cells are included is able to float due to the buoyancy in the culture medium, and, in a case where the semipermeable membrane 10 is provided on the top surface and the bottom surface as in the cell-culturing device 200, since the top surface is in contact with air and the bottom surface is in contact with the culture medium, it is possible to culture the cells in the gas phase and the liquid phase.

In addition, the support 12 may be fixed to the cell-culturing device 200 or may be detachable.

[Third Embodiment]

FIG. 4 is a perspective view schematically showing a cell-culturing device according to a third embodiment of the present invention.

A cell-culturing device 300 shown here is the same as the cell-culturing device 100 shown in FIG. 2 except for being provided with tubes 13. That is, the cell-culturing device 300 is provided with the semipermeable membrane 10 on the top surface and the bottom surface, and has the shape of a cylinder sealed on the side surface by the member 11. Furthermore, the tubes 13 are provided so as to face the outer surface of the cell-culturing device 300, the tubes 13 are inserted inside the cell-culturing device 300, and two of the tubes 13 and the cell-culturing device 300 are in communication.

Providing the tube 13 makes it possible for the cell-culturing device 300 to also supply the culture medium from the side surface. Furthermore, connecting the cell-culturing device 300 provided with the tubes 13 to each other makes it possible to construct the organ-type chip system described below.

In addition, an openable and closable device (not shown) such as a plug or a valve is preferably provided at the end of the opposite side to the side where the tube 13 is inserted into the cell-culturing device 300.

[Fourth Embodiment]

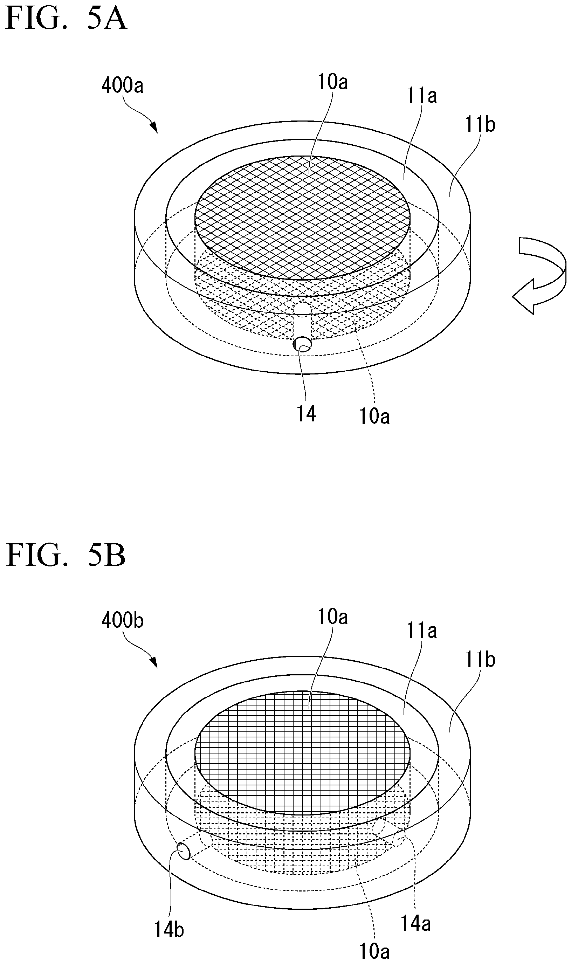

FIG. 5A and FIG. 5B are perspective views schematically showing a cell-culturing device according to a fourth embodiment of the present invention. In a cell-culturing device 400a shown in FIG. 5A, the inside and the outside of the device communicate with each other via an injection hole 14. On the other hand, a cell-culturing device 400b shown in FIG. 5B does not communicate between the inside and the outside of the device.

The cell-culturing devices 400a and 400b shown here is the same as the cell-culturing device 100 shown in FIG. 2 except that the semipermeable membrane is a semipermeable membrane 10a including the circular holding body 1, the injection hole 14 formed of a first injection hole 14a and a second injection hole 14b is provided, and the member is formed of a first member 11a and a second member 11b. That is, the cell-culturing devices 400a and 400b are provided with the semipermeable membrane 10a including the circular holding body 1 on the top surface and the bottom surface, and have the shape of a cylinder sealed on the side surface by the member 11. In addition, the member 11 is formed of the first member 11a and the second member 11b, and the second member 11b is provided so as to be in contact with the outer peripheral surface of the first member 11a. Furthermore, the first member 11a and the second member 11b are each provided with the first injection hole 14a and the second injection hole 14b penetrating the inner peripheral surface and the outer peripheral surface. Therefore, by rotating and adjusting the positions of the first member 11a and the second member 11b to the left and right, as shown in the cell-culturing device 400a, the first injection hole 14a and the second injection hole 14b are connected to make communication possible between the inside and the outside of the device. On the other hand, as shown in the cell-culturing device 400b, shifting the positions of the first injection hole 14a and the second injection hole 14b makes it possible to stop (interrupt) communication between the inside and the outside of the device.

The cell-culturing device 400a rotates the positions of the first member 11a and the second member 11b to the left and right to connect the first injection hole 14a and the second injection hole 14b and make communication possible between the inside and the outside of the device and also make it possible to supply the culture medium from the side surface. On the other hand, in the cell-culturing device 400b, the positions of the first member 11a and the second member 11b are rotated to the left and right to shift the positions of the first injection hole 14a and the second injection hole 14b and make it possible to stop (interrupt) communication between the inside and the outside of the device without providing an openable and closable device such as a plug or a valve.

[Fifth Embodiment]

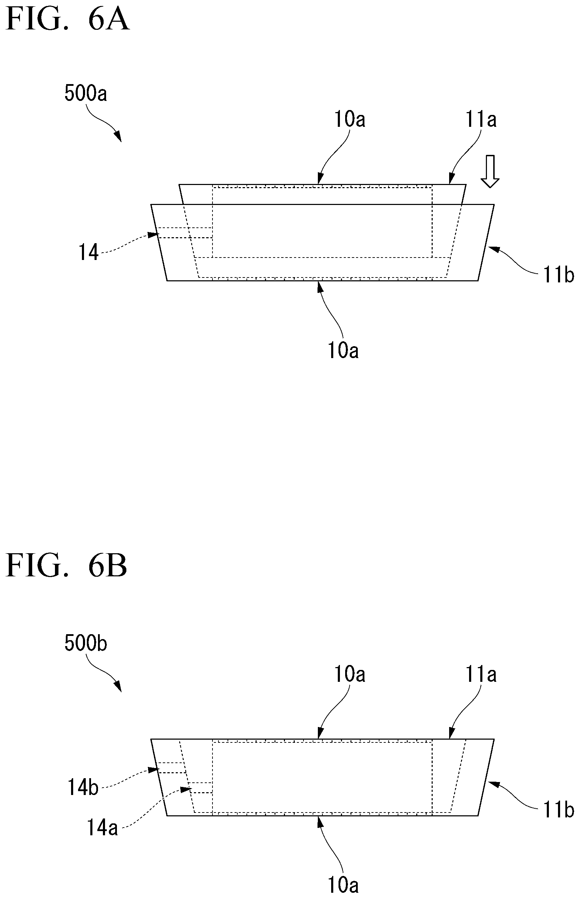

FIG. 6A and FIG. 6B are cross-sectional views schematically showing a cell-culturing device according to a fifth embodiment of the present invention. In a cell-culturing device 500a shown in FIG. 6A, the inside and the outside of the device communicate via the injection hole 14. On the other hand, in a cell-culturing device 500b shown in FIG. 6B, the inside and the outside of the device do not communicate.

The cell-culturing devices 500a and 500b shown here are the same as the cell-culturing device 100 shown in FIG. 2 except that the semipermeable membrane is the semipermeable membrane 10a including the circular holding body 1, the injection hole 14 formed of the first injection hole 14a and the second injection hole 14b is provided, and the member is formed of the first member 11a and the second member 11b. That is, the cell-culturing devices 500a and 500b are provided with the semipermeable membrane 10a including the circular holding body 1 on the top surface and the bottom surface, and have a cylindrical shape sealed on the side surface by the member 11. In addition, the member 11 is formed of the first member 11a and the second member 11b, and the second member 11b is provided so as to be in contact with the outer peripheral surface of the first member 11a. Furthermore, the first member 11a and the second member 11b are respectively provided with the first injection hole 14a and the second injection hole 14b penetrating the inner peripheral surface and the outer peripheral surface. Therefore, adjusting the positions of the first member 11a and the second member 11b up and down to connect the first injection hole 14a and the second injection hole 14b as shown in the cell-culturing device 500a makes it possible for the inside and the outside of the device to communicate with each other. On the other hand, as shown in the cell-culturing device 500b, shifting the positions of the first injection hole 14a and the second injection hole 14b up and down makes it possible to stop (interrupt) communication between the inside and the outside of the device.

The cell-culturing device 500a moves the positions of the first member 11a and the second member 11b up and down to connect the first injection hole 14a and the second injection hole 14b so as to make communication possible between the inside and outside of the device and also make it possible to supply the culture medium from the side surface. On the other hand, the cell-culturing device 500b moves the positions of the first member 11a and the second member 11b up and down to shift the positions of the first injection hole 14a and the second injection hole 14b so that it is possible to stop (interrupt) communication between the inside and the outside of the device without providing an openable and closable device such as a plug or a valve.

In addition, as shown in FIG. 6A and FIG. 6B, the shape of the engaging portions of the first member 11a and the second member 11b is preferably a tapered structure since the cell-culturing devices 500a and 500b are easy to operate up and down.

[Sixth Embodiment]

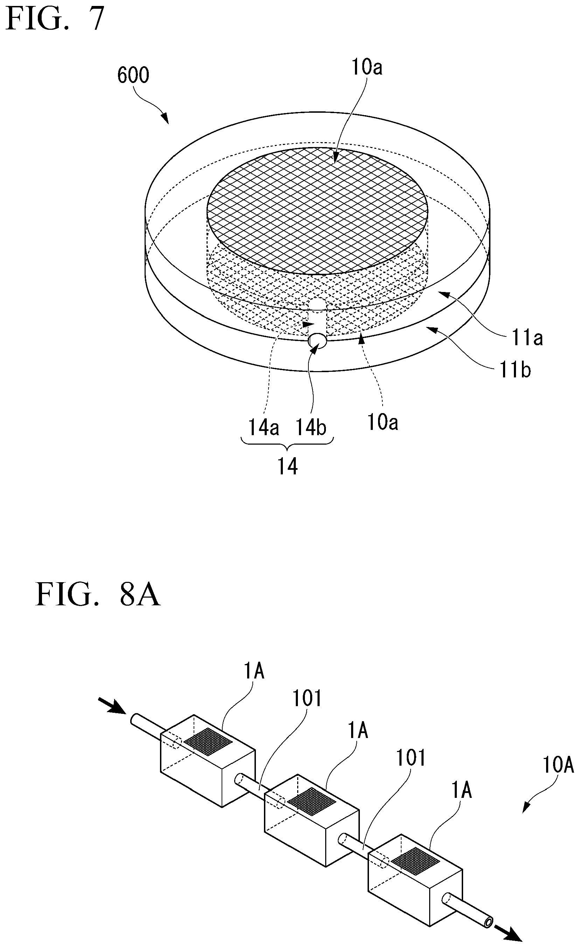

FIG. 7 is a perspective view schematically showing a cell-culturing device according to a sixth embodiment of the present invention.

A cell-culturing device 600 shown here is the same as the cell-culturing device 100 shown in FIG. 2 except that the semipermeable membrane is the semipermeable membrane 10a including the circular holding body 1, the injection hole 14 formed of the first injection hole 14a and the second injection hole 14b is provided, and the member is formed of the first member 11a and the second member 11b. That is, the cell-culturing device 600 is provided with the semipermeable membrane 10a including the circular holding body 1 on the top face and the bottom face, and has a cylindrical shape sealed on the side face by the member 11. In addition, the member 11 is formed of the first member 11a and the second member 11b, and the first member 11a and the second member 11b have the same shape. Furthermore, the first member 11a and the second member 11b are each provided with the first injection hole 14a and the second injection hole 14b which are semi-circular recesses on the top surface from the outer peripheral surface toward the center. Joining the first member 11a and the second member 11b using an adhesive or the like such that the top surfaces are in contact with each other and the first injection hole 14a and the second injection hole 14b are aligned with each other makes it possible to obtain the cell-culturing device 600 provided with the injection hole 14 penetrating from the outer peripheral surface to the inner peripheral surface shown in FIG. 7.

Providing the injection hole 14 also makes it possible for the cell-culturing device 600 to also supply the culture medium from the side surface.

In addition, an openable and closable device (not shown) such as a plug or a valve is preferably provided on the side in contact with the outside of the injection hole 14 of the cell-culturing device 600.

The cell-culturing device of the present embodiment is not limited to those devices shown in FIG. 2 to FIG. 7, but parts of the configurations shown in FIG. 2 to FIG. 7 may be changed or removed or other configurations may be added to the embodiments previously described, within a range in which the effect of the cell-culturing device of the present embodiment is not impaired.

For example, the cell-culturing device shown in FIG. 2 to FIG. 7, the member may have an open shape without being provided with a top surface.

In addition, for example, in the cell-culturing device shown in FIG. 2 to FIG. 4, the member may be provided with an injection hole. In a case where the injection hole is provided, it is preferable to provide a plug for closing the injection hole.

The shape of the injection hole is not particularly limited, and examples thereof include a circular shape, a polygonal shape (including regular polygonal shapes and the like), an elliptical shape, and the like.

It is possible to appropriately adjust the radius of the injection hole according to the thickness of the cell-culturing device (that is, the member height), and, for example, 10 μm or more and 1000 μm or less is possible.

In addition, the cell-culturing devices shown in FIG. 5A to FIG. 7 are shown with one injection hole being provided, but two or more may be provided.

In addition, for example, in the cell-culturing devices shown in FIG. 2 to FIG. 7, semipermeable membranes in which the top surface and the bottom surface include a holding body are shown, but the semipermeable membranes may be semipermeable membranes in which the top surface or bottom surface does not have a holding body. Having the semipermeable membrane including the holding body on either the top surface or the bottom surface makes it possible to more easily measure the number of cells.

In addition, for example, in the cell-culturing devices shown in FIG. 2 to FIG. 7, an adhesive layer may be provided between the semipermeable membrane and the member. In this case, the semipermeable membrane may be attachable to and detachable from the member via the adhesive layer. It is possible to make the semipermeable membrane attachable to and detachable from the outer surface of the semipermeable membrane by using an adhesive having low adhesion to the member and high adhesion to the semipermeable membrane. Due to this, after the cells are cultured using the cell-culturing device of the present embodiment, it is possible to easily take out the semipermeable membrane together with the cells in the device.

In addition, for example, in the cell-culturing devices shown in FIG. 2 to FIG. 7, a film having air-tightness may be provided on the outer surface of the semipermeable membrane via an adhesive layer. In this case, the film having air-tightness may be attachable to and detachable from the semipermeable membrane via the adhesive layer. It is possible to make a film having air-tightness attachable to and detachable from the outer surface of the porous film by using an adhesive having low adhesion to the film having air-tightness and having high adhesion to the porous membrane.

In addition, in the cell-culturing device of the present embodiment, it is possible to arbitrarily adjust the size and shape of each configuration (semipermeable membrane, member, and the like) according to the purpose.

<C onfigurations>

[Semipermeable Membrane]

Since the semipermeable membrane used in the cell-culturing device of the present embodiment has liquid-tightness in the gas phase, for example, in a case where a liquid such as a culture medium is included in the cell-culturing device of the present embodiment, it is possible to hold the liquid inside in the gas phase without leaking therefrom. This liquid-tightness is due to the surface tension on the semipermeable membrane. On the other hand, since it is possible for gas to pass therethrough, in a case where a liquid is included inside, the liquid inside evaporates over time.

In addition, since the semipermeable membrane used in the cell-culturing device of the present embodiment has a semipermeable property in the liquid phase, for example, in a case where the cell-culturing device of the present embodiment including the cells is immersed in a container including a culture medium, in the semipermeable membrane, the cells in the cell-culturing device do not pass to the outside of the device, while the nutrients dissolved in the culture medium are allowed to pass into the inside of the cell-culturing device, and it is possible for the cell products including the waste matter dissolved in the culture medium inside the cell-culturing device to pass to the outside of the cell-culturing device. Therefore, it is possible to use the cell-culturing device of the present embodiment for culturing cells over a long period of time.

More specifically, it is possible to set the semipermeable membrane used in the cell-culturing device of the present embodiment to allow a polymer compound having a molecular weight of about 1,000,000 or less to pass therethrough, for example, to allow a molecular compound having a molecular weight of about 200,000 or less to pass therethrough.

Examples of the material of the semipermeable membrane having the above properties include the same materials as exemplified in "Constituent Material" of "Semipermeable Membrane" described above.

[Member]

In the cell-culturing device of the present embodiment, it is possible to use a member having a liquid-tightness as a member forming a portion other than the semipermeable membrane. In addition, in the cell-culturing device of the present embodiment, the member forming the portion other than the semipermeable membrane may have air permeability or may not have air permeability.

In a case where the member has air permeability, it is possible to set the oxygen permeability coefficient to, for example, 100 $cm^3/m^2 \cdot 24$ hr $\cdot$ atm or more and 5000 $cm^3/m^2 \cdot 24$ hr $\cdot$ atm or less, for example, 1000 $cm^3/m^2 \cdot 24$ hr $\cdot$ atm or more and 3000 $cm^3/m^2 \cdot 24$ hr $\cdot$ atm or less, and, for example, 1200 $cm^3/m^2$ 24 hr atm or more and 2500 $cm^3/m^2$ 24 hr $\cdot$ atm or less. Furthermore, it is possible to set the carbon dioxide permeability coefficient to, for example, 1000 $cm^3/m^2 \cdot 24$ hr $\cdot$ atm or more and 20000 $cm^3/m^2 \cdot 24$ hr $\cdot$ atm or less, for example, 3000 $cm^3/m^2 \cdot 24$ hr $\cdot$ atm or more and 15000 $cm^3/m^2 \cdot 24$ hr $\cdot$ atm or less, and, for example, 5000 $cm^3/m^2 \cdot 24$ hr $\cdot$ atm or more and 10000 $cm^3/m^2 \cdot 24$ hr $\cdot$ atm or less.

In addition, in a case where the member does not have air permeability, it is possible to set the oxygen permeability coefficient to, for example, 100 $cm^3/m^2 \cdot 24$ hr $\cdot$ atm or less and, for example, 50 $cm^3/m^2 \cdot 24$ hr $\cdot$ atm or less. Furthermore, it is possible to set the carbon dioxide permeability coefficient to, for example, 1000 $cm^3/m^2 \cdot 24$ hr $\cdot$ atm or less and, for example, 500 $cm^3/m^2 \cdot 24$ hr $\cdot$ atm or less.

In the cell-culturing device of the present embodiment, as the material of a member forming a portion other than the semipermeable membrane, it is possible to use a material suitable for cell culturing. Examples of the material forming the portion other than the semipermeable membrane include glass materials, elastomer materials, plastics including dendritic polymers, copolymers, and the like, without being limited thereto.

Examples of the glass material include soda lime glass, Pyrex (registered trademark) glass, Vycor (registered trademark) glass, quartz glass, and the like.

Examples of the elastomer material include urethane rubber, nitrile rubber, silicone rubber, silicone resins (for example, polydimethylsiloxane), fluororubber, acrylic rubber, isoprene rubber, ethylene propylene rubber, chlorosulfonated polyethylene rubber, epichlorohydrin rubber, chloroprene rubber, styrene butadiene rubber, butadiene rubber, polyisobutylene rubber, and the like.

Examples of the dendritic polymer include poly(vinyl chloride), poly(vinyl alcohol), poly(methyl methacrylate), poly(vinyl acetate-co-maleic anhydride), poly(dimethylsiloxane) monomethacrylate, cyclic olefin polymer, fluorocarbon polymer, polystyrene, polypropylene, polyethyleneimine, and the like.

Examples of the copolymer include poly(vinyl acetate-co-maleic anhydride), poly(styrene-co-maleic anhydride), poly(ethylene-co-acrylic acid), derivatives thereof, and the like.

The material of the member may be formed of one type of material among the materials exemplified above or may be formed of two or more types.

In addition, in a case where the member is formed of two or more types of the materials exemplified above, the member may be formed of a mixture of the materials exemplified above. Alternatively, the member may be formed by combining members formed of one type of material, and the materials forming each member may be different from each other.

In addition, it is possible to appropriately select the shape of the member according to the entire shape of the cell-culturing device of the present embodiment and the portion forming the cell-culturing device of the present embodiment.

(Method of Producing Member)

It is possible to produce the member in the present embodiment using a known method depending on the material to be used.

For example, in a case where an elastomer material or plastic is used as the material of the member, examples of the method of producing the member include a compression molding method, an injection molding method, an extrusion molding method, and the like, without being limited thereto.

In addition, in a case where a glass material is used as the material of the member, examples of the production method include a droplet molding method, the Danner method, an overflow method, a float method, a blow molding method, a press molding method, and the like, without being limited thereto.

In addition, in a case of producing a member provided with an injection hole, a member provided with an injection hole may be produced by forming an injection hole by laser irradiation or the like after producing the member. Alternatively, a member provided with an injection hole may be produced by joining two members having the same shape and having a semi-circular recess in a portion corresponding to the injection hole. Alternatively, after a part of the member is made to protrude, an injection hole may be formed in the protruding portion.

[Plug]

In the cell-culturing device of the present embodiment, in a case where the member is provided with the injection hole, the embedded type plug to be used is preferably a material harder than the member. Specific examples thereof include metals such as iron and stainless steel and the like, without being limited thereto. In addition, in a case where the injection hole is provided in the protruding portion of the member, the plug to be used may be an embedded type or a covering type plug.

In addition, it is possible for the shape of the plug to be a shape capable of blocking the injection hole, and, in a case of an embedded type, examples thereof include a spherical shape, a conical shape, a truncated conical shape, a pyramidal shape, a truncated pyramidal shape, a cylindrical shape, a prismatic shape, or the like, and in the case of a covering type, examples thereof include cap shapes such as a spherical shell shape, a dome shape, a conical tubular shape, a conical cylinder shape, a cylindrical shape, a pyramidal tubular shape, a pyramidal cylindrical shape, and a square tubular shape, without being limited thereto.

[Support]

The material of the support used in the cell-culturing device of the present embodiment may be, for example, an organic material or an inorganic material.

Examples of the organic material include polyamide (such as nylon), polyolefm resin, polyester resin, polystyrene resin, polycarbonate, polyamide resin, silicone resin, and the like, without any particular limitation.

Examples of the inorganic material include ceramics, glass, and the like, without any particular limitation.

The shape of the support may be, for example, a sheet shape, a rod shape, and the like, without being limited thereto.

(Method of Producing Support)

It is possible to produce the support in the present embodiment by a known method depending on the material to be used.

For example, in a case where an organic material is used as the material of the support, examples of the production method include a compression molding method, a calender molding method, an injection molding method, an extrusion molding method, inflation molding, and the like, without being limited thereto.

In addition, examples of a production method in a case of using glass as a material of a support include the same methods as exemplified in (Method for Producing Member) described above.

In addition, examples of a production method in a case of using ceramics as a material of a support include a dry molding method (for example, a mold forming method, a cold isostatic pressing method, a hot pressing method, a hot isostatic pressing method, and the like), a plastic molding method (for example, a wheel molding method, an extrusion molding method, or an injection molding method), a cast molding method (for example, a slip casting method, a pressure casting method, a rotary casting method, or the like), a tape molding method, and the like, without being limited thereto.

[Tube]

The material of the tube used in the cell-culturing device of the present embodiment is not particularly limited and may be the above material having biocompatibility or a material suitable for culturing cells. Examples of the material having biocompatibility include the same materials as those exemplified in "Others" of "Constituent Materials" of "Semipermeable Membrane" described above. Examples of materials suitable for culturing the cells include the same materials as exemplified in "Member" described above.

In addition, more specifically, examples of suitably used tubes include catheters for medical use, catheters for indwelling needles, and the like.

(Method of Producing Tube)

It is possible to produce the tube in the present embodiment by a known method depending on the material to be used.

As a specific production method, it is possible to form a tubular shape using the same method as the method exemplified in "Method of Producing Semipermeable Membrane" of "Semipermeable Membrane" and "Method of Producing Member" of "Member" described above.

[Film having Air-Tightness]

It is possible to set the thickness of the film having air-tightness to, for example, 10 $\mu$m or more and 500 $\mu$m or less, for example, 30 $\mu$m or more and 300 $\mu$m or less, for example, and 50 $\mu$m or more and 150 $\mu$m or less.

Here, "the thickness of film having air-tightness" means the thickness of the entire film having air-tightness, and, for example, the thickness of the film having air-tightness formed of a plurality of layers means the total thickness of all the layers forming the film having air-tightness.

The material of the film having air-tightness may be any material having air-tightness.

Specifically, the material of the film having air-tightness may be an organic material or an inorganic material.

Examples of the organic material include polyamide (such as nylon, for example), polyolefin resin, polyester resin, polystyrene resin, polycarbonate, polyamide resin, silicone resin, and the like, without particular limitation.

Examples of the inorganic material include ceramics, glass, and the like, without particular limitation.

The film having air-tightness may be formed of one type of the materials exemplified above, or may be formed of two or more types.

In addition, in a case where the film having air-tightness is formed of two or more types of the materials exemplified above, the film having air-tightness may be formed of a mixture of the materials exemplified above. Alternatively, the film having air-tightness may be formed by laminating two or more films having air-tightness formed of one type of material, and the materials forming each film having air-tightness may be different from each other.

(Method for Producing Film Having Air-tightness)

It is possible to produce the film having air-tightness by a known method depending on the material to be used.

Examples of a production method in a case of using an organic material as a material of the film having air-tightness include a compression molding method, a calender molding method, an injection molding method, an extrusion molding method, inflation molding, and the like, without being limited thereto.

In addition, examples of the production method in a case of using glass as the material of the film having air-tightness include the same method as exemplified above (method of producing a member).

In addition, for example, examples of a production method in a case of using ceramics as a material of a film having air-tightness include a dry molding method (for example, a mold forming method, a cold isostatic pressing method, a hot pressing method, a hot isostatic pressing method, and the like), a plastic molding method (for example, a wheel molding method, an extrusion molding method, or an injection molding method), a cast molding method (for example, a slip casting method, a pressure casting method, a rotary casting method, or the like), a tape molding method, and the like, without being limited thereto.

<Method of Producing Cell-Culturing Device>

It is possible to produce the cell-culturing device of the present embodiment by assembling only a semipermeable membrane or a semipermeable membrane and a member so as to have a desired shape. In addition, as necessary, it is possible to provide a support and a tube.

The production methods of each of the semipermeable membrane, the member, the support, and the tube are as described above.

More specifically, a detailed description will be given below of the method of producing the cell-culturing device of the present embodiment shown in FIG. 2. First, two semipermeable membranes 10 are prepared having the same size as the top surface and the bottom surface of the member 11 or one size larger than the top surface and the bottom surface of the member 11. Next, the prepared semipermeable membranes 10 are joined so as to be the top surface and the bottom surface of the member 11, respectively.

Examples of the method of joining the semipermeable membrane 10 and the member 11 include a joining method using an adhesive, a joining method with a double-sided tape, a joining method by heat welding using a heat sealer or a hot plate, ultrasonic waves, a laser, or the like, a method using tenon and mortise joining by producing a tenon and a mortise (for example, single-sided, double-sided, three-sided, four-sided, small rooted, marginal, two-tenon, two-step tenon, or the like), and the like, without being limited thereto. In addition, one of these joining methods may be used, or two or more types may be used in combination.

In addition, the adhesive may be any adhesive which has no cytotoxicity, and may be an adhesive of a synthetic compound or an adhesive of a natural compound.

Examples of the adhesive of the synthetic compound include urethane adhesive, cyanoacrylate adhesive, polymethyl methacrylate (PMMA), calcium phosphate adhesive, and resin-based cement, and the like.

Examples of the adhesive of the natural compound include fibrin glue, gelatin glue, and the like.

In addition, as the double-sided tape, it is possible to use double-sided tape which is not cytotoxic, and tape or the like used in medical applications is preferably used. Specific examples thereof include tapes having a structure in which a pressure-sensitive adhesive layer is laminated on both sides of a support, and examples of the pressure-sensitive adhesive layer include known rubber-based, acryl-based, urethane-based, silicone-based, or vinyl ether-based pressure-sensitive adhesives or the like. More specific examples thereof include double-sided adhesive tape for skin application (product numbers: 1510, 1504 XL, 1524, and the like) produced by 3M Japan Ltd., double-sided adhesive tape for skin (product numbers: ST 502, ST 534, and the like) produced by Nitto Denko Corporation, double-sided medicinal tape (product numbers: #1088, #1022, #1010, #809 SP, #414125, #1010 R, #1088 R, #8810 R, #2110 R, and the like) produced by Nichiban Medical Corp., thin foam material double-sided adhesive tape (product numbers: #84010, #84015, #84020, and the like) produced by DIC Corp., and the like.

Using double-sided adhesive tapes of different colors such as black and white (for example, #84010 WHITE, #84010 BLACK, and the like produced by DIC Corporation) on the top surface and bottom surface of the member, respectively, makes it possible to easily distinguish the top surface side and the bottom surface side by visual observation in a case where the semipermeable membrane is transparent or translucent.

Next, it is possible to obtain the cell-culturing device 100 by carrying out sterilization using γ-line irradiation, electron beam irradiation, UV irradiation, ethylene oxide gas (EOG) or the like, and adjusting the size of the semipermeable membrane 10 or the member 11 as necessary.

Alternatively, as a method for producing the cell-culturing device of the present embodiment shown in FIG. 2, for example, first, the semipermeable membrane and the member are joined using a semipermeable membrane not including a holding body and the same method as the production method described above and the cell-culturing device is produced. Next, the cell-culturing device may be produced by adhering and drying the holding body to the member using the adhesive described above.

In addition, in a case where the support 12 is provided as in the cell-culturing device 200 of the present embodiment shown in FIG. 3, the support 12 may be joined in advance to the semipermeable membrane 10 or the member 11, in addition, the support 12 may be joined to the assembled cell-culturing device 200. The joining method may carry out the fixing using the same method as the method of joining the semipermeable membrane and the member described above, or may enable detachable attachment using a fastener or the like.

In addition, in a case where a tube is provided as in the cell-culturing device 300 of the present embodiment shown in FIG. 4, the tube 13 may be inserted in advance into the semipermeable membrane 10 or the member 11, or the tube 13 may be inserted into the assembled cell-culturing device 300. As a tube insertion method, for example, in a case where a catheter of an indwelling needle is used as a tube, it is possible to insert the tube by inserting the indwelling needle into the cell-culturing device and then pulling out the inner needle.

«Method of Using Cell-Culturing Device»

As described below, it is possible to use the cell-culturing device of the present embodiment for, for example, cell culturing, cell transporting, tissue-type chips, organ-type chips, organ-type chip systems, and the like.

In the present specification, "tissue" refers to a unit of a structure gathered in a pattern based on a certain lineage in which one type of stem cell is differentiated, and which has a single role as a whole. For example, in epidermal keratinocytes, stem cells existing in the basal layer of the epidermis are differentiated into cells forming the granular layer through the spinous layer and are terminally differentiated to form a stratum corneum so as to exhibit a barrier function as the epidermis. Thus, constructing a multicellular structure including one type of cells derived from one cell lineage makes it possible for the tissue-type chip of the present embodiment to reproduce, for example, epithelial tissue, connective tissue, muscle tissue, nerve tissue, and the like.

In addition, in the present specification, an "organ" is formed of two or more types of tissues and has one function as a whole. Thus, constructing a multicellular structure including at least two types of cells having different cell lineages makes it possible for the organ-type chip of the present embodiment to reproduce, for example, a stomach, intestines, a liver, a kidney, and the like.

Furthermore, in the present specification, "organ system" refers to a group of two or more organs having similar functions and a group of two or more organs having a series of functions as a whole. Thus, by combining a plurality of tissue-type chips or organ-type chips, it is possible for the organ-type chip system of the present embodiment to reproduce, for example, organ systems such as a digestive system, a cardiovascular system, a respiratory system, a urinary system, a reproductive system, an endocrine system, a sensory organ system, a nervous system, an exerciser system, and a nervous system. Living bodies maintain homeostasis by interactions between these organ systems. In the organ-type chip system of the present embodiment, since it is possible to combine a plurality of different organ-type chips of the organ system, it is also possible to analyze the interaction between different organs of the organ system. For example, in an organ-type chip system in which a small intestine-type chip, a liver-type chip, and a neural-type chip are connected in this order, in a case where a drug is added to the small intestine-type chip, the drug absorbed by the small intestine-type chip is metabolized by the liver-type chip, and it is possible to analyze the toxicity and the like exerted by the liver metabolites of the drug excreted by the liver-type chip on the neural-type chip.

<Cell-Culturing Method>

In one embodiment, the present invention provides a cell-culturing method using the cell-culturing device described above.

According to the culturing method of the present embodiment, it is possible to easily culture cells and construct a multicellular structure. In addition, it is possible to maintain cells for approximately 3 to 30 days, and to maintain cells for a longer period than in the related art. Furthermore, according to the culturing method of the present embodiment, it is possible to obtain the tissue-type chip described below.

A detailed description will be given below of the culturing method of the present embodiment.

First, a culture medium in which cells are suspended is prepared. Next, using a pipette, a dropper, or a nozzle such as an injection needle (including a winged needle, an indwelling needle, or the like) or the like, the suspension is injected into the cell-culturing device described above.

In a case where the cell-culturing device has an injection hole, injection of the cell suspension is performed from the injection hole, but in a case of using an injection needle, injection may be performed from the injection hole, or injection may be performed by directly piercing the member.

In addition, it is preferable to close the injection hole with an embedded type plug formed of a material having higher hardness and lower elasticity than the material of the member after injection of the culture medium in which the cells are suspended. In addition, in a case where the injection hole is provided in the protruding portion of the member, it is preferable to close the injection hole with an embedded type or a cover type plug.

More specifically, for example, in a case where the injection site is a member formed of plastic, the injection hole may be closed with a stainless steel ball or the like.

Next, the cell-culturing device into which the culture medium in which the cells are suspended is injected is able to carry out the culturing in at least one of a gas phase and a liquid phase and construct a multicellular structure. It is possible to perform the culturing in the gas phase, for example, using a container such as an empty petri dish, and it is possible to carry out the culturing in a time period in which the cells do not dry and die.

In addition, the culturing in the liquid phase may be carried out using a container such as a petri dish including a culture medium, for example. Alternatively, it is possible to perform stirring and culturing using, for example, a spinner flask or the like. Due to this, it is possible to carry out culturing in a plurality of cell-culturing devices into which a culture medium in which cells are suspended is injected at the same time, and to easily obtain a large amount of multicellular structures in a short period of time.

In addition, the culturing in the gas phase and the liquid phase may be performed, for example, by floating the cell-culturing device on a container such as a petri dish including the culture medium using the cell-culturing device having the support shown in FIG. 3.

Examples of the cells used in the culturing method of the present embodiment include vertebrate cells such as mammalian cells, avian cells, reptile cells, amphibian cells, and fish cells; invertebrate cells such as insect cells, crustacean cells, molluscan cells, and protozoal cells; bacteria such as gram-positive bacteria (for example, Bacillus species), and gram-negative bacteria (for example, Escherichia coli or the like); yeasts, plant cells, small living organisms formed of single cells or a plurality of cells, and the like.

Examples of the small living organisms include unicellular organisms such as amoeba, paramecium, closterium, pinnularia, chlorella, euglena, and phacus; microcrustceans such as daphnia, artemia larvae, copepods, ostracoda, thecostraca larvae, phyllocarida shrimp larvae, peracarida shrimp larvae, and eucarida shrimp larvae; planaria (including regenerating planaria after fine cutting), terrestrial arthropod larvae, nemathelminthes, plant seeds (in particular, germinated seeds), callus, protoplast, marine microorganisms (for example, marine bacteria such as vibrio, pseudomonas, eromonas, alteromonas, flavobacterium, cytophaga, and flexibacter, algae such as cyanobacteria, cryptophytes, dinoflagellates, diatom, raphidophytes, golden algae, haptophytes, euglenophytes, prasinophyceae, green algae, or the like), larval fish, larval shellfish, and the like, without being limited thereto.

For example, in a case where germinating seeds are cultured using the cell-culturing device of the present embodiment, since the top surface of the cell-culturing device has a hardness which is sufficient to allow germinated buds to penetrate and is formed of biodegradable material, germinated seeds placed in the device are able to be directly implanted in soil to allow plants to grow.

In the present specification, "biodegradable material" means a material having a property of being decomposed into inorganic matter by microorganisms or the like in soil or water.

Examples of vertebrate cells (in particular, mammalian cells) include reproductive cells (sperm, eggs, or the like), somatic cells, stem cells, and progenitor cells forming a living body, cancer cells separated from a living body, cells (cell lines) separated from a living body and stably maintained outside by being immortalized, cells separated from a living body and artificially genetically modified, cells separated from a living body and with the nuclei artificially exchanged, and the like, without being limited thereto. In addition, multicellular globular aggregates (spheroids) of these cells may also be used. In addition, a small tissue piece separated from normal tissue or cancer tissue of a living body may be used as it is in the same manner as a multicellular aggregate.

Examples of somatic cells forming a living body include cells collected from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood, heart, eye, brain, and nerve tissue, without being limited thereto. More specifically, examples of somatic cells include fibroblasts, bone marrow cells, immune cells (for example, B lymphocytes, T lymphocytes, neutrophils, macrophages, monocytes, or the like), red blood cells, platelets, osteocytes, bone marrow cells, pericytes, dendritic cells, epidermal keratinocytes (keratinocytes), adipocytes, mesenchymal cells, epithelial cells, epidermal cells, endothelial cells, vascular endothelial cells, lymphatic endothelial cells, hepatocytes, islet cells (for example, $\alpha$ cells, $\beta$ cells, $\delta$ cells, $\varepsilon$ cells, PP cells, or the like), chondrocytes, cumulus cells, glial cells, neural cells (neurons), oligodendrocytes, microglia, astrocytes, cardiomyocytes, esophageal cells, muscle cells (for example, smooth muscle cells, skeletal muscle cells, or the like), melanocytes, mononuclear cells, and the like, without being limited thereto.

A stem cell is a cell which combines the ability to replicate itself and the ability to differentiate into cells of a plurality of other lines. Examples of stem cells include embryonic stem cells (ES cells), embryonic tumor stem cells, embryonic reproductive stem cells, induced pluripotent stem cells (iPS cells), neural stem cells, hematopoietic stem cells, mesenchymal stem cells, hepatic stem cells, pancreatic stem cells, muscle stem cells, reproductive stem cells, intestinal stem cells, cancer stem cells, hair follicle stem cells, and the like, without being limited thereto.

A progenitor cell is a cell in the stage of being differentiated from the stem cell into a specific somatic cell or reproductive cell.

A cancer cell is a cell derived from a somatic cell and acquiring infinite proliferative capacity and is a malignant neoplasm which invades or causes metastasis in the surrounding tissue. Examples of cancers from which cancer cells are derived include breast cancer (for example, invasive ductal breast cancer, non-invasive ductal breast cancer, inflammatory breast cancer, and the like), prostate cancer (for example, hormone-dependent prostate cancer, hormone-independent prostate cancer, and the like), pancreatic cancer (for example, pancreatic duct cancer, and the like), stomach cancer (for example, papillary adenocarcinoma, mucinous adenocarcinoma, adenosquamous cancer, and the like), lung cancer (for example, non-small cell lung cancer, small cell lung cancer, malignant mesothelioma, and the like), colon cancer (for example, gastrointestinal stromal tumors, and the like), rectal cancer (for example, gastrointestinal stromal tumors, and the like), colorectal cancer (for example, familial colorectal cancer, hereditary non-polyposis colon cancer, gastrointestinal stromal tumors, and the like), small bowel cancer (for example, non-Hodgkin's lymphoma, gastrointestinal stromal tumors, and the like), esophageal cancer, duodenal cancer, tongue cancer, pharyngeal cancer (for example, nasopharyngeal cancer, oropharyngeal cancer, hypopharyngeal cancer, and the like), head and neck cancer, salivary gland cancer, brain tumors (for example, pineal gland astrocytoma, pilocytic astrocytoma, diffuse astrocytoma, anaplastic astrocytoma, and the like), neurinoma, liver cancer (for example, primary liver cancer, extrahepatic bile duct cancer, and the like), kidney cancer (for example, renal cell cancer, transitional epithelial cancer of renal pelvis and ureter, and the like), gallbladder cancer, pancreatic cancer, endometrial cancer, cervical cancer, ovarian cancer (for example, epithelial ovarian cancer, extragonadal germ cell tumors, ovarian germ cell tumors, low grade ovarian tumors, and the like), bladder cancer, urethral cancer, skin cancer (for example, intraocular (ocular) melanoma, Merkel cell cancer, and the like), angioma, malignant lymphoma (for example, reticulosarcoma, lymphosarcoma, Hodgkin's disease, and the like), melanoma (malignant melanoma), thyroid cancer (for example, medullary cancer of the thyroid, and the like), parathyroid cancer, nasal cancer, paranasal sinus cancer, bone tumors (for example, osteosarcoma, Ewing's tumor, uterine sarcoma, soft tissue sarcoma, and the like), metastatic medulloblastoma, angiofibroma, protruding dermal fibrosarcoma, retinal sarcoma, penile cancer, testicular tumors, pediatric solid cancer (for example, Wilms tumor, pediatric renal tumors, and the like), Kaposi's sarcoma, Kaposi's sarcoma caused by AIDS, maxillary sinus tumors, fibrous histiocytoma, leiomyosarcoma, rhabdomyosarcoma, chronic myeloproliferative disease, leukemia (for example, acute myelogenous leukemia, acute lymphoblastic leukemia, and the like) and the like, without being limited thereto.

In addition, in the present specification, the Chinese character for "cancer" is used to indicate a diagnosis name and the Japanese characters for "cancer" are used to represent a generic term for a malignant neoplasm.

A cell line is a cell which acquired infinite proliferative capacity due to artificial manipulation *in vitro*. Examples of cell lines include HCT116, Huh7, HEK293 (human embryonic kidney cells), HeLa (human cervical cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns 0/1, Jurkat, NIH3T3, PC12, S2, Sf9, Sf21, High Five, Vero, and the like, without being limited thereto.

As the culture medium used in the culturing method of the present embodiment, in a case where the cells are animal cells, it is possible to use a basic culture medium including components necessary for the cell survival and growth (inorganic salts, carbohydrates, hormones, essential amino acids, non-essential amino acids, vitamins) and the like, and the culture medium is able to be appropriately selected according to the type of cells. Examples of the culture medium include DMEM, Minimum Essential Medium (MEM), RPMI-1640, Basal Medium Eagle (BME), Dulbecco's Modified Eagle's Medium: Nutrient Mixture F-12 (DMEM/F-12), Glasgow Minimum Essential Medium (Glasgow MEM), and the like, without being limited thereto.

In addition, for a culture medium of bacteria, yeasts, plant cells, and small living organisms formed from single cells or a plurality of cells, a culture medium having a composition suitable for growth in each case may be prepared.

In addition, in the culturing method of the present embodiment, a component derived from an extracellular matrix, a physiologically active substance, and the like may be mixed and injected into a culture medium in which cells are suspended.

Examples of the component derived from an extracellular matrix include the same examples as exemplified for "Others" of "Constituent Material" of "Semipermeable Membrane" described above.

In addition, examples of physiologically active substances include cell growth factors, differentiation-inducing factors, cell adhesion factors, and the like, without being limited thereto. For example, by including a differentiation-inducing factor, in a case where the cells to be injected are stem cells, precursor cells, or the like, it is possible to induce differentiation of the stem cells or the precursor cells and to construct a multicellular structure reproducing a desired tissue.

In addition, in the culturing method of the present embodiment, the culture medium in which the cells are suspended may be injected so as to fill the capacity of the cell-culturing device, or an amount less than the capacity of the cell-culturing device may be injected. For example, in a case where the cell-culturing device has a structure in which a semipermeable membrane is provided on the top surface and the bottom surface as shown in FIG. 2 and the material of the semipermeable membrane is collagen, a culture medium in which the cells are suspended is injected with an injection needle or the like in an amount less than the capacity of the cell-culturing device and the needle or the like is pulled out such that the top surface and the bottom surface of the cell-culturing device are depressed using reduced pressure, and the cells are sandwiched between the semipermeable membrane on the top surface and the semipermeable membrane on the bottom surface, and it is possible to perform sandwich culturing using collagen.

In the culturing method of the present embodiment, it is possible to appropriately select the culture conditions depending on the type of cells to be cultured.

The culturing temperature may be, for example, 25°C or more and 40°C or less, for example, 30°C or more and 39°C or less, and, for example, 35°C or more and 39°C or less.

In addition, the $CO_2$ concentration during the culturing may be, for example, a condition of approximately 5% $CO_2$.

It is possible to appropriately select the culturing time according to the type of cells, the number of cells and the like, and the culturing time may be, for example, 3 days or more and 30 days or less, for example, 5 days or more and 20 days or less, and, for example, 7 days or more and 15 days or less.

<Cell Number Measurement Method>

In one embodiment, the present invention provides a method for measuring the number of cells using the cell-culturing device described above.

According to the measurement method of the present embodiment, it is possible to easily measure the number of cells included in the cell-culturing device without using a hemocytometer.

A detailed description will be given below of the measurement method of the present embodiment.

First, a culture medium in which cells are suspended is prepared. Next, using a pipette, a dropper, or a nozzle such as an injection needle (including a winged needle, an indwelling needle, or the like), a culture medium in which cells are suspended is injected into the cell-culturing device described above.

Examples of the cells used in the measurement method of the present embodiment include the same cells exemplified in "Cell-Culturing Method" described above. In addition, examples of the culture medium used in the measurement method of the present embodiment include the same solutions as exemplified in "Cell-Culturing Method" described above.

Next, the injection hole is closed with a plug and mixing is carried out such that the cells are uniformly dispersed in the cell-culturing device, and then the cell-culturing device is set in the microscope. Next, cells included in one square of the lattice formed by the holding body are measured. Next, for example, in a case where a lattice in which squares have a length and a width of 250 $\mu$m is formed in a holding body, using a lattice of 16 squares of 4 squares × 4 squares (that is, a lattice with 16 squares of 4 squares x 4 squares formed in 1 mm$^2$ with a length of 1 mm x a width of 1 mm), the cells included in each square are measured in each of the 16 squares, and the total number of cells in the 16 squares is calculated. The number of cells is measured at least once or more using 16 squares (1 mm$^2$) at different positions, and the average value of the number of cells included in 1 mm$^2$ is calculated. Then, substituting the average value of the number of cells into "N" of equation [1] makes it possible to calculate the number of cells "C" per 1 cm$^2$.

$$C = N \times 10^2 \ ... \ [1]$$

In equation [1], "10$^2$" means a converted capacity value with respect to 1 cm$^2$.

Furthermore, substituting the bottom area of the cell-culturing device into "S" of equation [2] makes it possible to calculate all the numbers of cells "A" included in the cell-culturing device.

$$A = C \ x \ S \ ... \ [2]$$

Measuring the number of cells makes it possible to culture the cells in the cell-culturing device until the desired number of cells is reached. Furthermore, using a reagent that stains only dead cells such as Trypan blue makes it possible to measure the viability of the cells in the cell-culturing device without taking the cells out of the cell-culturing device.

<Tissue-Type Chip>

In one embodiment, the present invention provides a tissue-type chip provided with the cell-culturing device described above including one type of cells.

The tissue-type chip of the present embodiment is able to be used as a substitute for a culture model or animal experiments in the related art for the screening of candidate drugs for various diseases or an evaluation test system for kinetics and toxicity of chemical substances including candidate drugs with respect to normal tissues, without the need to construct a culture model from scratch.

Furthermore, the culture models of the related art had a time restriction and had to be used immediately after construction, whereas it is possible to culture the tissue-type chip of the present embodiment for a long period of time.

Examples of the cells included in the tissue-type chip of the present embodiment include the same cells as exemplified in "Cell-Culturing Method" described above. In addition, it is possible to appropriately select the type of cells to be included depending on the type of tissue to be constructed.

In addition, the cells included in the tissue-type chip of the present embodiment may be in a middle stage of constructing the multicellular structure, or may be after the multicellular structure is constructed. It is possible to culture the tissue-type chip of the present embodiment for a long period of time of approximately 3 to 21 days even after the included cells have constructed the multicellular structure.

The density of cells included in the tissue-type chip of the present embodiment changes depending on the type of tissue to be constructed, but is preferably $2.0 \times 10^3$ cells/mL or more and $1.0 \times 10^9$ cells/mL or less, and more preferably $2.0 \times 10^5$ cells/mL or more and $1.0 \times 10^8$ cells/mL or less.

When the cell density is within the above range, it is possible to obtain a tissue-type chip having a cell density closer to that of living tissue.

It is possible to produce the tissue-type chip of the present embodiment using the method described in "Cell-Culturing Method" described above. In addition, it is possible for the maintaining conditions of the tissue-type chip after producing to be the same conditions as the culturing conditions described in "Cell-Culturing Method" described above. In addition, the inside of the tissue-type chip may include a culture medium or a gas such as air, and may not include a culture medium or a gas such as air. In a case where the tissue-type chip does not include a culture medium or a gas such as air, cells, or cells and components derived from an extracellular matrix are closely adhered, and a multicellular structure with a structure closer to that of tissues in a living body is constructed.

<Organ-Type Chip>

In one embodiment, the present invention provides an organ-type chip provided with a cell-culturing device as described above including at least two types of cells.

The organ-type chip of the present embodiment is able to be used as a substitute for a culture model or animal experiments in the related art for the screening of candidate drugs for various types of diseases or an evaluation test system for kinetics and toxicity of chemical substances including candidate drugs with respect to normal organs, without the need to construct a culture model from scratch.

Furthermore, the culture models of the related art had a time restriction and had to be used immediately after construction, whereas it is possible to culture the organ-type chip of the present embodiment for a long period of time.

Examples of the cells included in the organ-type chip of the present embodiment include the same cells as exemplified in "Cell-Culturing Method" described above. In addition, the type of cells to be included may include at least two types of cells, and it is possible to appropriately select the type of cells according to the type of the organ to be constructed.

In addition, the cells included in the organ-type chip of the present embodiment may be in a middle stage of constructing the multicellular structure, or may be after the multicellular structure is constructed. It is possible to culture the organ-type chip of the present embodiment for a long period of time of approximately 3 to 21 days even after the included cells have constructed the multicellular structure.

The density of cells included in the organ-type chip of the present embodiment changes depending on the type of the organ to be constructed, but is preferably $2.0 \times 10^3$ cells/mL or more and $1.0 \times 10^9$ cells/mL or less, and more preferably $2.0 \times 10^5$ cells/mL or more and $1.0 \times 10^8$ cells/mL or less.

When the cell density is within the above range, it is possible to obtain organ-type chips having a cell density closer to that of organs in a living body.

It is possible to produce the organ-type chip of the present embodiment using the method described in "Cell-Culturing Method" described above. In addition, it is also possible for the maintaining conditions of the organ-type chip after producing to be the same conditions as the culturing conditions described in "Cell-Culturing Method" described above. In addition, the inside of the organ-type chip may include a culture medium or a gas such as air, or may not include a culture medium or a gas such as air. In a case where a culture medium or a gas such as air is not included in the organ-type chip, cells, or cells and components derived from an extracellular matrix are closely adhered, and a multicellular structure with a structure closer to that of organs in a living body is constructed.

<Kit for Providing Multicellular Structure>

In one embodiment, the present invention provides a kit for providing a multicellular structure, which is provided with an openable and closable sealed container including the tissue-type chip described above or the organ-type chip described above, and a culture medium.

The kit of the present embodiment is able to be used as a substitute for a culture model or animal experiments in the related art for the screening of candidate drugs for various types of diseases or an evaluation test system for kinetics and toxicity of chemical substances including candidate drugs with respect to normal tissues and organs, without the need to construct a culture model from scratch.

Furthermore, the culture models of the related art had a time restriction and had to be used immediately after construction, whereas it is possible to carry out culturing with the kit of the present embodiment for a long period of time.

The cells included in the tissue-type chip or in the organ-type chip in the kit of the present embodiment may be in a middle stage of constructing the multicellular structure, or may be after the multicellular structure is constructed. Among these, since it is possible to use such cells immediately for an *in vitro* test system, the cells included in the tissue-type chip or the organ-type chip in the kit of the present embodiment are preferably after the multicellular structure is constructed.

It is possible to use an openable and closeable sealed container in the kit of the present embodiment, without particular limitation. Examples of the sealed container include a conical tube with a screw cap, a flask for cell culturing with a screw cap, a bag with a zipper, a bag with a chuck, and the like, without being limited thereto.

As a material of the sealed container, it is possible to use a sealed container having liquid-tightness. In addition, the sealed container may have air permeability or may not have air permeability. More specifically, examples of the material of the sealed container include materials which are the same as exemplified in "Member" described above. Among these, as a material of the sealed container, plastic is hard to break and is lightweight, which is preferable.

It is possible to appropriately select the culture medium in the kit of the present embodiment depending on the type of cells included in the tissue-type chip or the organ-type chip and specific examples thereof include those exemplified in "Cell-Culturing Method" described above.

In addition, in the kit of the present embodiment, the culture medium is preferably included to the full capacity of the sealed container. Pouring the culture medium in the sealed container to full capacity and sealing the container prevents drying of tissue-type chips or organ-type chips and makes it possible to safely carry the tissue-type chips or organ-type chips.

In the kit of the present embodiment, the number of tissue-type chips or organ-type chips included in the sealed container may be one or two or more. In a case of being two or more, a tissue-type chip or an organ-type chip in which the same type of multicellular structure is constructed is preferable.

The kit of the present embodiment may further be provided with a culture medium separately from the culture medium included in the sealed container. The culture medium may be of the same type as included in the sealed container or may be another type. By separately providing a culture medium, it is possible to use as a substitute culture medium for culturing tissue-type chips or organ-type chips until the kit of the present embodiment is used in an *in vitro* test system or the like.

<Organ-Type Chip System>

In one embodiment, the present invention provides an organ-type chip system which is provided with at least two of the tissue-type chips as described above or the organ-type chips as described above, in which the tissue-type chips or the organ-type chips are connected while maintaining a sealing property.

The organ-type chip system of the present embodiment can be expected to be used as a substitute for a culture model or animal experiments in the related art for the screening of candidate drugs for various types of diseases or an evaluation test system for kinetics and toxicity of chemical substances including candidate drugs with respect to a plurality of normal tissues and organs, without the need to construct a culture model from scratch, and the like.

In the present specification, "sealed" means a closed state without gaps.

[First Embodiment]

FIG. 8A is a perspective view schematically showing the organ-type chip system according to the first embodiment of the present invention.

An organ-type chip system 10A shown here has a structure in which three tissue-type chips 1A are each connected via a tube 101.

For example, allowing the culture medium to flow from the direction of the arrow on the left side to the direction of the arrow on the right side makes it possible to carry out the culturing in a state where the three tissue-type chips 1A are connected. In addition, for example, allowing candidate drugs for various diseases to flow from the direction of the arrow on the left side in the direction of the arrow on the right side makes it possible to verify the drug efficacy against the disease, the metabolic pathway of the drug and metabolites thereof, the cytotoxicity, and the like.

The tissue-type chip 1A shown in FIG. 8A is the same as described in "Tissue-Type Chip" described above. It is possible to appropriately select the type of cells (not shown) forming the multicellular structure constructed in the tissue-type chip 1A according to the type of the desired organ or organ system.

The tube 101 shown in FIG. 8A is the same as the tube 13 in FIG. 4, and the configuration thereof is the same as described in "Tube" described above.

[Second Embodiment]

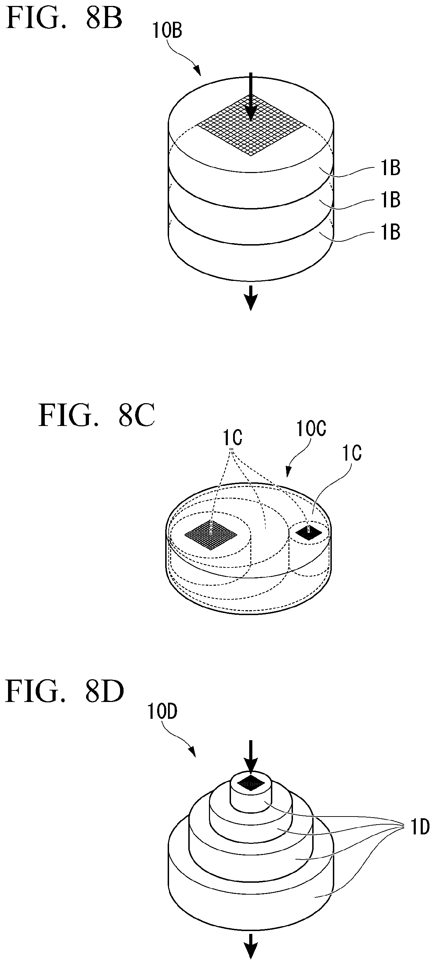

FIG. 8B is a perspective view schematically showing the organ-type chip system according to the second embodiment of the present invention.

An organ-type chip system 10B shown here has a structure in which three tissue-type chips 1B having the same size are laminated. At this time, at least the top surface and the bottom surface of each tissue-type chip 1B are semipermeable membranes.

For example, allowing a culture medium to flow from the direction of the arrow on the upper side in the direction of the arrow on the lower side makes it possible to carry out the culturing in a state where the three tissue-type chips 1B are laminated. In addition, for example, allowing candidate drugs for various diseases to flow from the direction of the arrow on the upper side in the direction of the arrow on the lower side makes it possible to verify the drug efficacy against the disease, the metabolic pathway of the drug and metabolites thereof, the cytotoxicity, and the like.

[Third Embodiment]

FIG. 8C is a perspective view schematically showing an organ-type chip system according to a third embodiment of the present invention.

An organ-type chip system 10C shown here has four tissue-type chips 1C of different sizes, and has a structure in which the small tissue-type chip 1C is sealed in the largest tissue-type chip 1C. At this time, at least the top surface and the bottom surface of the largest tissue-type chip 1C are semipermeable membranes, and the tissue-type chip 1C sealed in the largest tissue-type chip 1C is a semipermeable membrane on the whole surface.

For example, it is possible to carry out culturing by placing the organ-type chip system 1C in a container such as a petri dish including a culture medium. In addition, for example, placing the organ-type chip system 1C in a container such as a petri dish including a candidate drug for various diseases makes it possible to verify the drug efficacy against the disease, the metabolic pathway of the drug and metabolites thereof, the cytotoxicity, and the like.

[Fourth Embodiment]

FIG. 8D is a perspective view schematically showing the organ-type chip system according to the fourth embodiment of the present invention.

An organ-type chip system 10D shown here is a structure in which four tissue-type chips 1D having different sizes are laminated from the bottom in order from the largest. At this time, at least the top surface and the bottom surface of each tissue-type chip 1D are semipermeable membranes.

For example, allowing the culture medium to flow from the direction of the arrow on the upper side in the direction of the arrow on the lower side makes it possible to carry out the culturing in a state where the four tissue-type chips 1D are laminated. In addition, for example, allowing a candidate drug for various diseases to flow from the direction of the arrow on the upper side in the direction of the arrow on the lower side makes it possible to verify the drug efficacy against the disease, the metabolic pathway of the drug and metabolites thereof, the cytotoxicity, and the like.

The organ-type chip system according to the present embodiment is not limited to FIGS. 8A to 8D and a part of the configurations shown in FIGS. 8A to 8D may be changed or deleted or another configuration may be added to what has been described so far within a range that does not impair the effect of the organ-type chip system of the present embodiment.

For example, in FIG. 8A to FIG. 8D, a case where a tissue-type chip is provided is exemplified, but an organ-type chip may be provided at least in part.

For example, the organ-type chip system shown in FIG. 8A may be provided with an openable and closeable device, such as a plug or a valve, for each tube.

In addition, in the organ-type chip system shown in FIG. 8B and FIG. 8D, each tissue-type chip may have a support and further, in order to fix each tissue-type chip, the outer periphery of the top surface and the bottom surface may be fixed with an adhesive or the like.

In addition, in the organ-type chip system of the present embodiment, it is possible to arbitrarily adjust the size and shape of each configuration (tissue-type chip, tube, and the like) according to the purpose.

The organ-type chip system of the present embodiment itself is able to reproduce organs such as the liver, stomach, intestines, and the like. Furthermore, combining a plurality of the organ-type chip systems of the present embodiment makes it possible to reproduce organ systems such as the digestive system, the cardiovascular system, the respiratory system, the urinary system, the reproductive system, the endocrine system, the sensory organ system, the nervous system, the exerciser system, the nervous system, and the like.

Examples

A description will be given below of the present invention with reference to Examples, but the present invention is not limited to the following Examples.

[Production Example 1] Production of semipermeable membrane 1

1. Preparation of Holding Body

First, a polyester mesh (T80-70; produced by Yamani Inc., line diameter: 70 $\mu$m, mesh size: 248 $\mu$m $\times$ 248 $\mu$m) was used as a holding body and cut into a circular shape with a diameter of 34 mm with scissors. Next, 70% ethanol was added to a 60 mm diameter petri dish (BD Falcon, Cat #351007). Next, a circular mesh having a diameter of 34 mm to be used as a holding body was immersed in this petri dish for approximately 10 minutes and sterilized. Next, 70% ethanol was removed from the petri dish, 5 mL of PBS (SIGMA, D 8537) was added instead, and the holding body was washed. This washing with PBS was repeated three times in total. After washing, one holding body was transferred to a 35 mm diameter petri dish (BD Falcon, Cat #353001), and 2 mL of DMEM containing 10% FBS, 20 mM HEPES, 100 units/mL penicillin, and 100 $\mu$g/mL streptomycin (may be referred to below as "culture medium") was added thereto and immersion was carried out for 10 minutes.

2. Production of Semipermeable Membrane 1

For the production of the semipermeable membrane, a holding body-embedded Native Collagen Vitrigel (registered trademark) membrane (content per unit area of native collagen: 0.5 mg/cm$^2$) was prepared (may be referred to below as "semipermeable membrane 1") based on a known method (reference literature: Japanese Unexamined Patent Publication No. 8-228768). Two of the semipermeable membranes 1 were produced.

(1) First, 3 mL of culture medium was added to an ice-cooled 50 mL conical tube, 3 mL of a 0.5% collagen acidic solution for cell culturing I-AC (produced by Koken Co., Ltd.) was added thereto and mixed uniformly to prepare a 0.25% collagen sol.

(2) Next, the culture medium was removed from a 35 mm diameter petri dish containing one holding body, and then 2 mL of 0.25% collagen sol was poured therein. Next, the mixture was allowed to stand for 2 hours in a 37°C cell culture incubator in the presence of 5% $CO_2$/95% air to form a gel and prepare a native collagen gel in which the holding body was embedded.

(3) The native collagen gel in which the holding body was embedded was transferred into a simple clean bench installed in a thermo-hygrostat at 10°C and a humidity of 40% (40% RH). Thereafter, the result was allowed to stand for 2 days and dried to obtain a native collagen gel dried body embedded with the holding body.

(4) Next, the result was removed from the thermo-hygrostat, 2 mL of PBS was poured into a 35 mm diameter petri dish containing a native collagen gel dried body in which the holding body was embedded, and the result was allowed to stand for 10 minutes and rehydrated. After removal of the PBS, 2 mL of PBS was poured therein again and allowed to stand for 10 minutes, then the native collagen Vitrigel (registered trademark) membrane in which the rehydrated holding body was embedded was detached from the bottom of the petri dish, transferred into a new 35 mm diameter petri dish in which 2 mL of PBS was poured, and equilibrated with PBS.

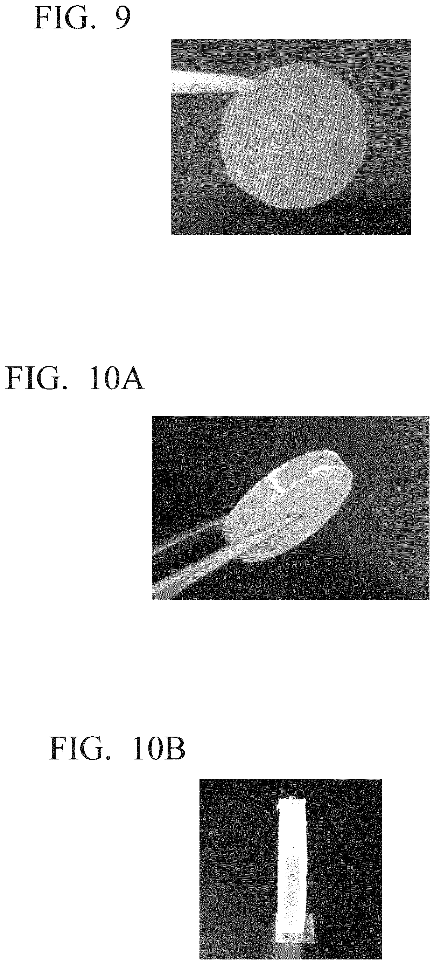

(5) Next, the obtained native collagen Vitrigel (registered trademark) membrane in which the holding body was embedded was placed on a 96 × 96 × 15 mm petri dish (Azuwan D-210-16) on which vinyl was laid, transferred into a simple clean bench installed in a thermo-hygrostat at 10°C and a humidity of 40% (40% RH), and dried again to obtain a native collagen Vitrigel (registered trademark) membrane dried body in which the holding body was embedded. A native collagen Vitrigel (registered trademark) membrane dried body in which the holding body was embedded was cut into a circular shape with a diameter of 13 mm with scissors, and the holding body-embedded collagen Vitrigel (registered trademark) membrane dried body (semipermeable membrane 1) was obtained (refer to FIG. 9).

[Production Example 2]

1. Production of Semipermeable Membrane (Control)

(1) First, a nylon membrane (Amersham Pharmacia Cat #RPN 1782 B) was hollowed out with a hollowing machine (produced by Morishita Seihan Ltd., blade: diameter: 24 mm to 33 mm) to prepare a cyclic nylon membrane support having an outer diameter of 33 mm and an inner diameter of 24 mm. Next, 70% ethanol was added to a 60 mm diameter petri dish (BD Falcon, Cat #351007). The cyclic nylon membrane support was immersed in this petri dish for approximately 10 minutes and sterilized. Next, 70% ethanol was removed from the petri dish, 5 mL of PBS (SIGMA, D 8537) was added instead, and the cyclic nylon membrane support was washed. This washing with PBS was repeated three times in total. After washing, one cyclic nylon membrane support was transferred to a 35 mm diameter petri dish (BD Falcon, Cat #353001), 2 mL of the culture medium was added thereto, and immersion was carried out for 10 minutes.

(2) Using the same method as in "2. Production of Semipermeable Membrane 1" of Production Example 1 except that the cyclic nylon membrane support was embedded instead of the holding body, a native collagen Vitrigel (registered trademark) membrane dried body in which the cyclic nylon membrane support was enclosed (may be referred to as "semipermeable membrane (control)") was obtained.

[Production Example 3] Production of Semipermeable Membrane 2

1. Preparation of Holding Body

A polyester mesh (T80-70; produced by Yamani Inc., line diameter: 70 μm, mesh size: 248 μm × 248 μm) was used as a holding body and cut into a circular shape with a diameter of 13 mm with scissors. First, 70% ethanol was added to a 35 mm diameter petri dish (BD Falcon, Cat #351008). Next, the circular mesh with a diameter of 13 mm to be used as a holding body was immersed in this petri dish for approximately 10 minutes and sterilized. Next, 70% ethanol was removed from the petri dish, 2 mL of PBS (SIGMA, D 8537) was added instead, and the holding body was washed. This washing with PBS was repeated three times in total. After washing, PBS was removed, 2 mL of culture medium was added thereto and immersion was carried out for 10 minutes.

2. Production of Semipermeable Membrane 2

For the production of the semipermeable membrane, using 0.25% collagen sol as an adhesive in accordance with a known method (reference document: Japanese Unexamined Patent Application Publication No. 2015-203018), a holding body was pinched and joined between two native collagen Vitrigel (registered trademark) membranes (content per unit area of native collagen: 0.5 mg/cm$^2$) to prepare the semipermeable membrane (may be referred to below as "semipermeable membrane 2")). Two of the semipermeable membranes 2 were produced.

(1) First, a semipermeable membrane (control) was prepared using the same method as in "1. Production of Semipermeable Membrane (Control)" in Production Example 2.

(2) Next, the two semipermeable membranes (control) were rehydrated. One rehydrated semipermeable membrane (control) was placed on a 96 × 96 × 15 mm petri dish (Azuwan #D-210-16) on which vinyl was laid, and a holding body which is a 13 mm diameter circular mesh immersed in the culture medium was placed in the center thereof, and 0.1 mL of 0.25% collagen sol prepared using the same method as in "2. Production of semipermeable membrane 1 (1)" of Production Example 1 was added thereto. Next, another rehydrated semipermeable membrane (control) was covered thereon, and the holding body was sandwiched by two semipermeable membranes (control) rehydrated so as to not contain air bubbles. Next, the result was transferred into a simple clean bench installed in a thermo-hygrostat at 10°C and a humidity of 40% (40% RH), dried overnight, and to obtain a dried body in which the holding body was sandwiched and adhered between two semipermeable membranes (control).

(3) The dried body with the holding body sandwiched and adhered between two semipermeable membranes (control) was transferred into FUNA-UV-Linker (Funakoshi Co., Ltd.: FS-1500/15 W/254 nm) and irradiated with UV at an irradiation dose of 200 mJ/cm$^2$. Next, the dried body with the holding body sandwiched and adhered between the two semipermeable membranes (control) was flipped over and irradiated one more time with UV at an irradiation dose of 200 mJ/cm$^2$. By UV irradiation of a total of 400 mJ/cm$^2$, even when rehydrated, it was possible to maintain the adhesion without separating two semipermeable membranes (control).

(4) A dried body of a semipermeable membrane (control) in which a holding body was sandwiched and adhered was cut out into a circular shape with a diameter of 13 mm with scissors to obtain a collagen Vitrigel (registered trademark) membrane material dried body (semipermeable membrane 2) in which a holding body is sandwiched and adhered.

[Example 1]

1. Production of Cell-culturing device

By attaching a semipermeable membrane including a holding body to both surfaces of an acrylic resin ring (outer diameter 13 mm, inner diameter 7.98 mm, thickness 2.0 mm, and injection hole diameter 0.7 mm), the following three types of cell-culturing devices were produced. In addition, as a control, a cell-culturing device was produced in which only a semipermeable membrane was attached to both surfaces of an acrylic resin ring without a holding body. In the present Example, the semipermeable membrane including the holding body was attached to both surfaces of the acrylic resin ring, but the semipermeable membrane including the holding body was attached to only one surface of the acrylic resin ring and a semipermeable membrane without a holding body may be attached on the other side.

(1) Production of Cell-culturing device 1

First, a polyurethane adhesive was applied to one surface of an acrylic resin ring, and the semipermeable membrane 1 produced in Production Example 1 was adhered thereto. Next, a polyurethane adhesive was similarly applied to the other surface of the acrylic resin ring which was flipped over to attach the semipermeable membrane 1 produced in Production Example 1 (may be referred to below as "cell-culturing device 1").

(2) Production of cell-culturing device 2

First, a polyurethane adhesive was applied to one surface of an acrylic resin ring to attach the semipermeable membrane 2 produced in Production Example 3. Next, a polyurethane adhesive was similarly applied to the other surface of the acrylic resin ring which was flipped over to attach the semipermeable membrane 2 produced in Production Example 3 (may be referred to below as "cell-culturing device 2").

(3) Production of Cell-culturing device (Control)

(3-1) First, after rehydrating the two semipermeable membranes (control) produced in Production Example 2, after clamping between two ring-shaped rubber magnets, fixing with magnetic force, and then re-drying according to a known method (reference document: Japanese Unexamined Patent Application Publication No. 2007-185107), and thereby a dried body of a wrinkle-free semipermeable membrane (control) was obtained.

(3-2) Next, a polyurethane adhesive was applied to one surface of an acrylic resin ring, a dried body of a semipermeable membrane (control) clamped between the ring-shaped rubber magnets was attached, and then the ring-shaped rubber magnet was removed and a dried body of the semipermeable membrane (control) protruding from the acrylic resin ring was cut out with scissors. The same operation was also applied to the other side of the acrylic resin ring which was flipped over (may be referred to below as "cell-culturing device (control)").

(4) Production of Cell-culturing device 3

(4-1) First, a cell-culturing device (control) was obtained using the same method as in "(3) Production of Cell-culturing device (Control)" described above.
(4-2) Next, a polyester mesh (T80-70; produced by Yamani Inc., line diameter: 70 $\mu$m, mesh size: 248 $\mu$m $\times$ 248 $\mu$m) was used as a holding body, cut into a circular shape with a diameter of 13 mm with scissors, immersed in 70% ethanol for approximately 10 minutes to sterilize, and then dried.
(4-3) Next, a polyurethane adhesive was applied to only the ring region on both surfaces of an acrylic resin ring to which a semipermeable membrane (control) was attached, and a sterilized holding body of a 13 mm diameter circular mesh was attached thereto (may be referred to below as "cell-culturing device 3"). That is, the holding body on the outer side of the semipermeable membrane (control) adhered only at the outer peripheral portion (ring region).

2. Physical Properties of Membrane of Cell-culturing device

(1) Confirmation of Bending of Membrane of Cell-culturing device

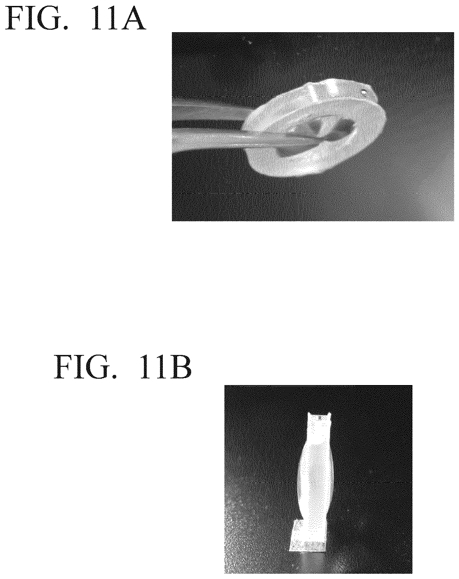

First, PBS was injected from each injection hole of the cell-culturing device 1, the cell-culturing device 2, the cell-culturing device 3, and the cell-culturing device (control), and the insides of the cell-culturing device 1, the cell-culturing device 2, the cell-culturing device 3, and the cell-culturing device (control) were filled with PBS, and the injection holes were closed using stainless steel balls. Next, the cell-culturing device 1, the cell-culturing device 2, the cell-culturing device 3, and the cell-culturing device (control) were left standing vertically. FIG. 10B and FIG. 11B show the state of the cell-culturing device 1 and the cell-culturing device (control), respectively.
From FIG. 10B, in the cell-culturing device 1 provided with the two semipermeable membranes 1, the membrane was smooth without bending even when filled with PBS. The same phenomenon was also confirmed for the cell-culturing device 2 and the cell-culturing device 3.
On the other hand, from FIG. 11B, in the cell-culturing device (control) provided with two semipermeable membranes (control), the filling with PBS made both of the membranes bend and bulge in a convex manner.
From the above, it was shown that in a cell-culturing device provided with a semipermeable membrane including a holding body, the membrane can be held without bending.

(2) Confirmation of Membrane Strength of Cell-culturing device

Next, for the cell-culturing device 1, the cell-culturing device 2, the cell-culturing device 3, and the cell-culturing device (control) filled with PBS, the surface of the semipermeable membrane was picked up using tweezers. FIG. 10A and FIG. 11A show states of the cell-culturing device 1 and cell-culturing device (control), respectively.
From FIG. 10A, in the cell-culturing device 1 provided with the two semipermeable membranes 1, it was possible to hold the membrane using tweezers without damage. The same phenomenon was also confirmed for the cell-culturing device 2 and the cell-culturing device 3.
On the other hand, from FIG. 11A, in the cell-culturing device (control) provided with two semipermeable membranes (control), the membrane was compressed by the tweezers and damaged.
From the above, it was shown that, in the cell-culturing device provided with the semipermeable membrane including the holding body, the membrane was held without damage using tweezers and was easy to handle.

[Test Example 1] Culturing of HepG2 Cells of Human Liver Cancer Cell Line using Cell-culturing device

(1) HepG2 cells cultured in advance (purchased from RIKEN BioResource Research Center, RCB 1648) were recovered and mixed with the culture medium to obtain a concentration of $1.0 \times 10^5$ cells/mL to prepare a suspension of HepG2 cells.
(2) Next, the suspension of HepG2 cells was filled in a 1 mL syringe with an indwelling needle. Next, the tip of the indwelling needle was injected through the injection holes of the cell-culturing device 1, the cell-culturing device 2, and the cell-culturing device 3 produced in Example 1. Next, 100 $\mu$L of a suspension of HepG2 cells was injected into the cell-culturing device 1, the cell-culturing device 2, and the cell-culturing device 3, and the injection holes were closed using stainless steel balls.
(3) Next, 1 mL of culture medium was injected into each well of a 24-well plate and each device in which HepG2 cells were cultured was submerged to start culturing.

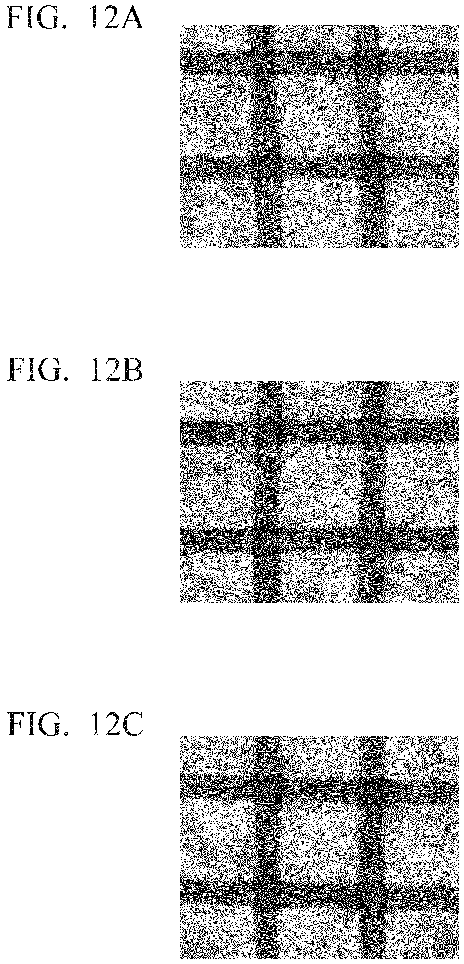

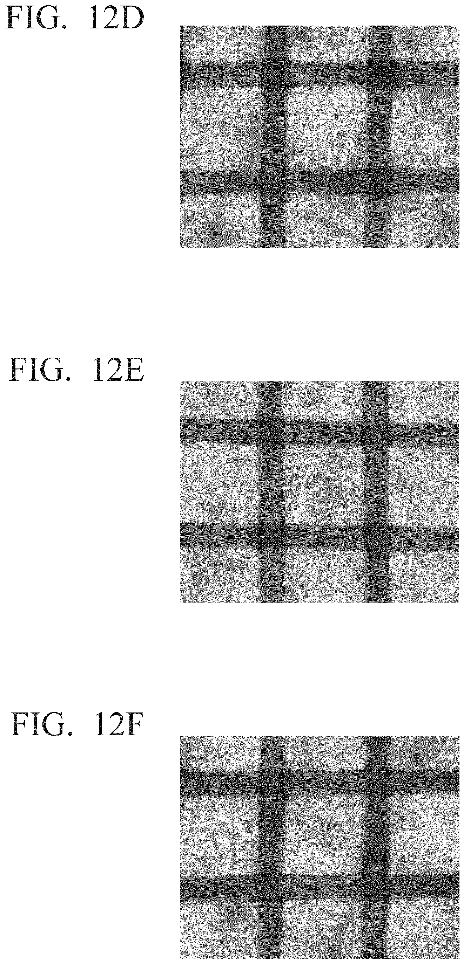

(4) Next, the culture medium was exchanged every other day and HepG2 cells sealed in the cell-culturing device were observed and photographed over time on the first day of culturing, the second day, the fourth day, the seventh day, the tenth day, and the fourteenth day, using a phase contrast microscope. The results of the cell-culturing device 3 are shown in FIG. 12A to FIG. 12F.

From FIG. 12A to FIG. 12F, it was shown that it is possible to measure cells included in one square of the lattice of the holding body using a phase contrast microscope until around the seventh day of culturing. The same phenomenon was confirmed also for the cell-culturing device 1 and the cell-culturing device 2.
In addition, it was shown that HepG2 cells proliferated over time, and that culturing is possible using the cell-culturing device.

Industrial Applicability

The semipermeable membrane of the present embodiment has a moderate strength which is difficult to bend and easy to handle. In addition, the cell-culturing device of the present embodiment not only has excellent cell protection performance but is also easy to handle, is able to carry out cell culturing for a long period of time, and makes it possible to measure the number of cells. Furthermore, by constructing a tissue-type chip, an organ-type chip, or an organ-type chip system using the cell-culturing device of the present embodiment can be expected to be used as a substitute for a culture model or animal experiments in the related art for a confirmation test or the like of a drug efficacy against disease, the metabolic pathway of the drug and metabolites thereof, the cytotoxicity, and the like.

Reference Signs List

1 HOLDING BODY
10 SEMIPERMEABLE MEMBRANE
10a SEMIPERMEABLE MEMBRANE INCLUDING HOLDING BODY
11 MEMBER
11a FIRST MEMBER
11b SECOND MEMBER
12 SUPPORT
13, 101 TUBE
14 INJECTION HOLE
14a FIRST INJECTION HOLE
14b SECOND INJECTION HOLE
100, 200, 300, 600 CELL-CULTURING DEVICE
400a, 500a CELL-CULTURING DEVICE (INSIDE AND OUTSIDE OF DEVICE COMMUNICATE VIA INJECTION HOLE)
400b, 500b CELL-CULTURING DEVICE (INSIDE AND OUTSIDE OF DEVICE DO NOT COMMUNICATE)
1A, 1B, 1C, 1D TISSUE-TYPE CHIP
10A, 10B, 10C, 10D ORGAN-TYPE CHIP SYSTEM

**Claims**

1. A semipermeable membrane, comprising:
a holding body with a low water absorption property having a lattice structure and having a semipermeable property in a liquid phase.

2. The semipermeable membrane according to Claim 1,
wherein the lattice structure of the holding body functions as a scale of micrometer units.

3. The semipermeable membrane according to Claim 1 or 2,
wherein the holding body is formed of polyester or polystyrene.

4. The semipermeable membrane according to any one of Claims 1 to 3, further comprising:
a material having biocompatibility.

5. The semipermeable membrane according to any one of Claims 1 to 4,
wherein a material having biocompatibility is a component derived from an extracellular matrix available for gelation.

6. The semipermeable membrane according to Claim 5,
wherein the component derived from the extracellular matrix available for gelation is native collagen or atelocollagen.

7. A cell-culturing device, comprising:
the semipermeable membrane according to any one of Claims 1 to 6 in at least a part thereof.

8. The cell-culturing device according to Claim 7, having liquid-tightness in a gas phase.

9. The cell-culturing device according to Claim 7 or 8, into which cells suspended in a culture medium are able to be injected and in which an internal volume is 10 mL or less.

10. The cell-culturing device according to any one of Claims 7 to 9,
wherein the whole device is formed of the semipermeable membrane.

11. A tissue-type chip, comprising:
the cell-culturing device according to any one of Claims 7 to 10, including one type of cells.

12. The tissue-type chip according to Claim 11, wherein a density of the cells is $2.0 \times 10^3$ cells/mL or more and $1.0 \times 10^9$ cells/mL or less.

13. An organ-type chip, comprising: the cell-culturing device according to any one of Claims 7 to 10, including at least two types of cells.

14. The organ-type chip according to Claim 13, wherein a density of the cells is $2.0 \times 10^3$ cells/mL or more and $1.0 \times 10^9$ cells/mL or less.

15. A kit for providing a multicellular structure, comprising: an openable and closable sealed container including the tissue-type chip according to Claim 11 or 12 or the organ-type chip according to Claim 13 or 14, and a culture medium.

16. An organ-type chip system, comprising: at least two of the tissue-type chips according to Claim 11 or 12 or the organ-type chips according to Claim 13 or 14, wherein the tissue-type chips or the organ-type chips are connected while maintaining a sealing property.

17. A cell-culturing method using the cell-culturing device according to any one of Claims 7 to 10.

18. A method for measuring a number of cells using the cell-culturing device according to any one of Claims 7 to 10.

# FIG. 1

# FIG. 2

# FIG. 3

# FIG. 4

# FIG. 5A

# FIG. 5B

FIG. 6A

500a

10a    11a

14    11b

10a

FIG. 6B

500b

10a    11a

14b    11b

14a

10a

FIG. 7

FIG. 8A

FIG. 8B

10B

1B
1B
1B

FIG. 8C

1C  10C  1C

FIG. 8D

10D

1D

FIG. 9

FIG. 10A

FIG. 10B

FIG. 11A

FIG. 11B

FIG. 12A

FIG. 12B

FIG. 12C

FIG. 12D

FIG. 12E

FIG. 12F

## INTERNATIONAL SEARCH REPORT

| International application No. |
| --- |
| PCT/JP2017/046650 |

**A. CLASSIFICATION OF SUBJECT MATTER**
Int.Cl. B01D69/06(2006.01)i, B01D69/10(2006.01)i, B01D71/08(2006.01)i,
C12M1/34(2006.01)i, C12M3/00(2006.01)i, C12N5/07(2010.01)i, C12Q1/06(2006.01)i

According to International Patent Classification (IPC) or to both national classification and IPC

**B. FIELDS SEARCHED**

Minimum documentation searched (classification system followed by classification symbols)
Int.Cl. B01D69/06, B01D69/10, B01D71/08, C12M1/34, C12M3/00, C12N5/07, C12Q1/06

| Documentation searched other than minimum documentation to the extent that such documents are included in the fields searched | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2013/011962 A1 (JAPAN SCIENCE AND TECHNOLOGY AGENCY) 24 January 2013, claims, paragraphs [0016], [0020], [0023], [0033], [0036], [0052], examples, fig. 1–16 & US 9139807 B2, claims, column 3, lines 20–34, column 4, line 37 to column 5, line 3, column 6, lines 20–24, column 7, lines 1–19, column 9, lines 13–46, examples & EP 2733199 A1 & CA 2842023 A1 & CN 103649302 A & KR 10–2014–0019475 A | 1–4, 7–10, 17–18<br>5–6, 11–16 |

☒ Further documents are listed in the continuation of Box C.    ☐ See patent family annex.

| \* | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 29 March 2018 (29.03.2018) | 10 April 2018 (10.04.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

International application No.

PCT/JP2017/046650

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 8-228768 A (RES DEV CORP OF JAPAN) 10 September 1996, claims, paragraphs [0001], [0007], [0009], [0019], fig. 1-7 (Family: none) | 5-6, 11-16 |
| X<br>Y | US 3929583 A (CANADIAN PATENTS AND DEVELOPMENT LIMITED) 30 December 1975, claims, column 3, line 1 to column 5, line 17, fig. 1-7 (Family: none) | 1-4, 7, 9-14, 17-18<br>5-6 |
| X<br>Y | WO 92/14838 A1 (NIHON MILLIPORE KOGYO KABUSHIKI KAISHA) 03 September 1992, claims, page 5, line 12 to page 8, line 18, fig. 1-3 & US 5811251 A, claims, column 3, line 49 to column 4, line 59, fig. 1-3 & JP 3133328 B2 & EP 529084 A1 & DE 69219963 T2 | 1-4, 7, 9-14, 17-185-6 |
| X | Microfilm of the specification and drawings annexed to the request of Japanese Utility Model Application No. 53507/1975 (Laid-open No. 133344/1976) (KURARAY CO., LTD.) 27 October 1976, claims, page 3, line 8 to page 4, line 7, fig. 1-4 (Family: none) | 1 |
| X | JP 7-299340 A (KUBOTA CORP.) 14 November 1995, claims, paragraph [0014], fig. 1-7 (Family: none) | 1 |
| X | JP 4-271817 A (FUJI PHOTO FILM CO., LTD.) 28 September 1992, claims, paragraph [0010], fig. 1-5 (Family: none) | 1 |
| X | JP 54-132291 A (KURARAY CO., LTD.) 15 October 1979 (Family: none) | 1-18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017006741 A
- JP 2012065555 A
- WO 2008130025 A
- JP 8228768 A
- JP 2001149763 A

- WO 2012026531 A
- JP 2012115262 A
- JP 2015035978 A
- JP 2015203018 A
- JP 2007185107 A